# EUROPEAN PATENT SPECIFICATION

(11) **EP 1 888 101 B1**
(45) Date of publication and mention of the grant of the patent: **21.03.2012**
(21) Application number: 06772251.2
(22) Date of filing: 06.06.2006
(51) Int. Cl.: A61K 38/22, A61K 38/17

(54) **COMPOSITIONS AND METHODS FOR LIPO MODELING**
ZUSAMMENSETZUNGEN UND VERFAHREN FÜR DIE LIPOMODELLIERUNG
COMPOSITIONS ET PROCEDES DE LIPOMODELAGE

(30) Priority: 06.06.2005 US 688271 P
(43) Date of publication of application: 20.02.2008
(60) Divisional application: 11154066.2
(73) Proprietor: Georgetown University, Washington, DC 20057 (US)
(72) Inventor: ZUKOWSKA, Zofia, Silver Spring, MD 20910 (US); KUO, Lydia, San Jose, CA 95129 (US); BAKER, Stephen, McLean, VA 22102 (US); JOHNSON, Michael, Rockville, MD 20853 (US); LEE, Edward, W., Irvine, CA 92604 (US)
(74) Representative: Gates, Marie Christina Esther
(86) International application number: PCT/US2006/021873
(87) International publication number: WO 2006/133160

(56) References cited:
- US-A1- 2004 115 135
- US-A1- 2004 214 855

## Description

### RELATED APPLICATION

This application claims the benefit of the filing date of U.S. provisional application having serial number 60/688,271 and entitled "COMPOSITIONS AND METHODS FOR LIPO MQDELING", filed on June 6, 2005.

### GOVERNMENT SUPPORT

This invention was made with government support under Grant Numbers R01 HL67357-02 and R03 DE016050-01 awarded by the National Institutes of Health. The government has certain rights in this invention.

### BACKGROUND

The role of soft tissue augmentation in plastic surgery includes both cosmetic and reconstructive applications. Cosmetic indications include filling fine wrinkles and deeper creases in the skin to alleviate the signs of aging. Liposuction is the most commonly performed cosmetic procedure in the United States, and the most common complication of liposuction is post-operative contour irregularity. In reconstructive surgery fat grafting is used to correct deformities from congenital anomalies, trauma, cancer, infections, disease, or side effects of medications (protease inhibitor-induced facial wasting). If a predictable way to add volume to the concavities was developed; there would be an enormous market. Additionally, if a method were developed to "melt away" the convexities of body contour or undesirable deposits of fat, the clinician could literally model the body similar to a sculptor modeling clay. Reconstructive indications include smoothing irregularities in reconstructed breasts or rebuilding facial volume in patients afflicted with congenital, traumatic, or neoplastic deformities. Such a therapy would be extremely effective as an adjunct or stand alone therapy in remodeling the fat and would also improve other metabolic parameters such as insulin resistance and other morbidities associated with obesity such as cardiovascular diseases or diabetes.

The ideal material for soft tissue augmentation has not been identified. Many alloplastic materials have been used and continue to be developed for use in plastic surgery. These include bovine collagen (Zyderm^{™}), human collagen

(Cosmoderm^{™}), hyaluronic acid (Restylane^{™}), crushed dermis, methylmethacrylate (Artefill^{™}), hydroxyapatite (Radiance^{™}), and numerous other materials. The alloplastic materials are expensive and their results frequently temporary. Those materials that provide permanent augmentation do not become physiologically incorporated into the body. A blood supply does not develop within the implanted material. The long term effects of these permanent fillers are unknown, but it is likely that they do not remodel with the aging tissue resulting in a material that may not be aesthetically acceptable as the patient's tissues undergo the normal physiologic changes associated with aging. The ideal material would be biocompatible, inexpensive, and abundant. For these reasons, autologous human fat is ideal. However, the use of autologous, fat is complicated by the unpredictability of its survival. Since the advent of molecular genetics and the discovery of the many bioactive growth factors, no study has evaluated their role in graft maintenance, and no one to date has found a reliable method of achieving reliable long-term survival of grafted human fat.

With the number of overweight individuals on the increase, there is a great need for methods that may be used to reduce fat deposits and treat or prevent diseases and conditions associated with excess body fat. Even in patients who are not obese, selective elimination of fat deposits is often indicated in reconstructive and cosmetic surgery. In reconstructive surgery fat grafting is used to correct deformities from congenital anomalies, trauma, cancer, infections, disease, or side effects of medications. Currently, various methods of liposuction are used to eliminate undesirable fat deposits. However; this is uncomfortable and frequently results in the need for IV sedation or general anesthesia. Furthermore, a great need exists for new methods that may be used for long term solutions for soft tissue augmentation.

### SUMMARY OF THE INVENTION

The present invention relates to a method of remodeling fat, such as in a human or other mammal, and compositions useful in the methods. This invention provides a non-to-minimally invasive therapy, such as bi-directional therapy for large- or small-scale reconstructive plastic surgery which comprises remodeling fat by 1) preventing resorption and/or inducing growth of (increase in) fat, for better survival of transplanted fat pads (e.g. craniomaxillofacial surgery). Inducing resorption and/or inhibiting fat deposition can also reduce adverse metabolic consequences of obesity by improving glucose tolerance and reducing insulin resistance.

As described herein, Applicants have discovered that a neurotransmitter, neuropeptide Y (NPY), is released from sympathetic nerves and used by the body to remodel its own adipose tissue. NPY, via its specific Y receptors, stimulates fat growth by directly stimulating preadipocyte proliferation and adipogenesis, and, indirectly, by increasing tissue vascularization. Furthermore, they have discovered that treatment with Y receptor antagonists reduces visceral fat and improves metabolic risk factors such as glucose tolerance, while Y receptor agonists increase it. Liposuction alone does not improve insulin action and risk factors for coronary heart disease and like other weight-management regimens, has the problem of recurrence and the rebound effect. In contrast, craniomaxillofacial reconstructive surgery is often plagued by the opposite problem of resorption of transplanted fat pads.

The method of the present invention can be used to improve the duration of effect, the predictability, and precision of reconstructive surgery by fat remodeling via a Y receptor agonist to stimulate fat deposit. The method of fat remodeling of the present invention can be used as an adjunct therapy to traditional weight loss techniques (e.g., anti-obesity drugs, diet and exercise, plastic surgery, etc.) or may also be used as a stand-alone therapy of local injections of slow release formulations of Y agonists and/or antagonists, where remodeling of smaller area is required. One of the benefits of the present method of liporemodeling is that site-specific application of either an agonist or an antagonist allows for fine-tuning of remodeling, e.g. appropriate and specific liporemodeling of adipose tissue. It can also be used for reconstruction of areas of the body, either alone or in combination with cosmetic or plastic surgery.

Also described herein use of a Y receptor agonist according to claim 1 in the manufacture of a medicament for increasing the stability of a fat graft in an individual. The Y receptor is, for example, a Y2 receptor, a Y5 receptor or a Y2/Y5 heterodimer. The Y receptor agonist can be any of the Y receptor agonists described herein. One or more than one (a combination of) agonists can be used. An agonist(s) can be combined with other drugs in the making of the medicament.

### BRIEF DESCRIPTION OF THE FIGURES

The foregoing and other features and advantages of the present invention will be more fully understood from the following detailed description of illustrative embodiments taken in conjunction with the accompanying drawings in which:
FIGURE 1: Immunohistochemistry of monoculture and coculture of murine 3T3-L1 preadipocytes. (A) Preadipocytes With negative oil red-O lipid staining (B) Preadipocytes cocultured with human microvascular endothelial cells (HMEC) stained for endothelial marker vWF (blue) and negative for oil red-O lipid staining (C) Preadipocytes cocultured with SKN-BE neuroblastoma cells (a sympathetic neuron model) stained for adrenergic neuron marker tyrosine hydroxylase (blue) and counterstained with eosin.
FIGURE 2: Immunohistochemistry of monoculture and coculture of SKN-BE neuroblastoma cells (adrenergic and NPY-containing sympathetic neuron-derived tumor cells). (A) Neuroblastoma with tyrosine hydroxylase (blue) and methyl green counterstain (B) Neuroblastoma with tyrosine hydroxylase (blue) cocultured with HMEC endothelial cells stained for vWF (red) (C) Preadipocytes cocultured with SKN-BE neuroblastoma cells stained for adrenergic neuron marker tyrosine hydroxylase (blue) and counterstained with eosin.
FIGURE 3: Immunohistochemistry of monoculture and coculture of HMEC endothelial cells. (A) Endothelial cells with endothelial marker vWF (red) (B) Endothelial cells with vWF (red) cocultured with Neuroblastoma with tyrosine hydroxylase (blue) (C) Endothelial cells with vWF (red) cocultured with preadipocytes counterstained with methyl green.
FIGURE 4: 3T3-L1 cell pellets stained for: (A) NPY (B) Y1R (C) Y2R (D) Y5R qualitatively confirmed results seen by quantitative RT-PCR. The presence of the Y5R was undetectable by RT-PCR, but was sparsely seen by immunocytochemistry.
FIGURE 5: In insulin preconditioned 3T3-L1 cells, synthetic NPY at 1x10-14 to 1x10-8 M stimulated differentiation into adipocytes containing lipid droplets stained by Oil red-O and secreting leptin.
FIGURE 6: Neuroblastoma-conditioned media causes proliferation of pre-adipocytes and endothelial cells while Y1/Y2/Y5R-antagonist cocktail blocks these effects.
FIGURE 7: (Top) Human fat pads injected subcutaneously into nude athymic mice (xenograft model) double-stained for cd31 (+) vessels (red) and negative TH (+) nerves (blue). Thin arrows represent small and large vessels in fat pads. (Bottom) Ultrasound images of fat pads with placebo- or NPY-pellet treatment. White arrows represent vacuolization of fat pads by ultrasound, confirmed by IHC (more numerous in placebo-treated than NPY-treated fat pads). White arrowheads represent areas of more dense necrotic tissue (more in NPY-treated than placebo-treated).
FIGURE 8: Increase of fat pad weight with NPY pellet insertion in WT and ob/ob mice but not eNOS-/-.
FIGURE 9: MRI of mice with thresholding for fat (represented by yellow) shows decreased fat in the region of the Y2R Antagonist pellet and increased fat in the region of the NPY pellet as compared to Placebo pellets.
FIGURE 10: Y2R-staining in subcutaneous abdominal fat pad of: (A) WT C57BL/6 (B) Stressed WT C57BL/6 (C) Stressed Y2KO (D) ob/ob + saline (E) ob/ob + Y2R antagonist (F) ob/ob + NPY; black arrows indicate Y2R-positive staining.
FIGURE 11: Density of von Willebrand factor positive vessels in abdominal fat of Y2R antagonist-treated ob/ob mice ** - p<0.01.
FIGURE 12: Quantitative real-time RT-PCR of subcutaneous fat pads from ob/ob mice shows a 70-fold increase in NPY mRNA levels over WT mice and a 7-fold increase in DPPIV mRNA over WT.
FIGURE 13: Fat thresholded volumetric rendered 3D-MRI were used to visualize increased fat deposits in C57BL/6, WT mice given a high fat diet (HF) and exposed to cold stress (ST) compared to stressed/high-fat diet Y2KO mice with fat deposits similar to nonstressed mice given standard chow. Obese ob/ob mice fed a standard chow diet showed the greatest amount of total body fat.
FIGURE 14: Plasma NPY levels were increased in stressed C57BL/6 WT and nonstressed ob/ob mice.
FIGURE 15: Core body temperature was elevated in ob/ob mice. Y2R-antagonist treatment resulted in a decrease in core body temperature in ob/ob mice with no change in WT mice.
FIGURE 16: SV129 WT and Y2KO mice were exposed to stress and given a high fat diet for two weeks. Visceral volume changes were calculated from 3D MRI images at week 2 and then mice were given either a Placebo pellet or a Y2 antagonist pellet.
FIGURE 17: C57BL/6 WT mice were placed in different treatment groups involving combinations of: cold stress (ST), high fat diet (HF), standard chow (SC), and Y2R antagonist (Y2Ant). Mice were evaluated by visceral volume changes calculated from 3D MRI images.
FIGURE 18: Plasma glucose levels were measured at times -30, 0, 30, 60, and 90 minutes after being given a glucose challenge. Impaired response to the challenge was seen in mice fed a high fat diet and stressed (Fig 18A) and in ob/ob obese mice (Fig 18B), which were both resolved with Y2R antagonist treatment (Fig 18A,B).

### DETAILED DESCRIPTION

Described herein are methods of remodeling or reshaping fat in humans and other mammals, as well as compositions for use in remodeling or reshaping fat. In specific embodiments,

Y receptor agonist(s) according to claim 1 are administered to stimulate fat deposition, such as in individuals who are in need of or desire augmentation of adipose tissue (e.g., breasts, hips, buttocks).

### Definitions

As used herein, the following terms and phrases shall have the meanings set forth below. Unless defined otherwise, all technical and scientific terms used herein have the same meaning as commonly understood to one of ordinary skill in the art.

The singular forms "a," "an," and "the" include plural reference unless the context clearly dictates otherwise.

The term "agent" is used herein to denote a chemical compound, a mixture of chemical compounds, a biological macromolecule (such as a nucleic acid, an antibody, a protein or portion thereof, e.g., a peptide), or an extract made from biological materials such as bacteria, plants, fungi, or animal (particularly mammalian) cells or tissues. The activity of such agents may render it suitable as a "therapeutic agent", which is a biologically, physiologically, or pharmacologically active substance (or substances) that acts locally or systemically in a subject.

The term "binding" refers to an association, which may be a stable association, between two molecules, e.g., between a polypeptide and a binding partner, due to, for example, electrostatic, hydrophobic, ironic and/or hydrogen-bond interactions under physiological conditions.

The terms "comprise" and "comprising" are used in the inclusive, open sense, meaning that additional elements may be included.

The term "including" is used to mean "including but not limited to". "Including" and "including but not limited to" are used interchangeably.

The term "mammal" is known in the art, and exemplary mammals include humans, primates, bovines, porcines, canine, felines, and rodents (e.g., mice and rates).

The term "pharmaceutically-acceptable salts" is art-recognized and refers to the relatively non-toxic, inorganic and organic acid addition salts of compounds, including, for example, those contained in compositions described herein.

The terms "nucleic acid" or "polynucleotide" refer to a polymeric form of nucleotides, including ribonucleotides and/or deoxyribonucleotides or a modified form of either type of nucleotide. The terms should also be understood to include, as equivalents, analogs of either RNA or DNA made from nucleotide analogs, and, as applicable to the embodiment being described, single-stranded (such as sense or antisense) and double-stranded polynucleotides.

The term "operably linked", when describing the relationship between two nucleic acid regions, refers to a juxtaposition wherein the regions are in a relationship permitting them to function in their intended manner. For example, a control sequence "operably linked" to a coding sequence is ligated in such a way that expression of the coding sequence is achieved under conditions compatible with the control sequences, such as when the appropriate molecules (e.g., inducers and polymerases) are bound to the control or regulatory sequence(s).

The term "PP-fold polypeptide" refers to a family of peptides sharing a similar structure called the PP-fold including pancreatic polypeptide (PP), peptide YY (PYY), and neuropeptide Y (NPY), and fragments, derivatives, variants, and analogs of the foregoing. Exemplary PP-fold polypeptides include, NPY, [Leu³¹, Pro³⁴]NPY, NPY₂₋₃₆, NPY₃₋₃₆, NPY₁₃₋₃₆, [D-Trp³²]NPY, [D-Arg²⁵]-NPY, [D-His²⁶]-NPY, Des-^{AA11-18}[Cys^{7,21}, D-Lys⁹(Ac), D-His²⁶, Pro³⁴]-NPY, [Phe⁷, Pro³⁴]-pNPY, C2-NPY, Cyclo S-S [Cys²⁰, Cys²⁴]pNPY, [D-Trp³²]NPY, [Ala³¹, Aib³²]-NPY, p[D-Trp³⁴]-NPY, PP, [cPP¹⁻⁷, NPY¹⁹⁻²³, His³⁴]-hPP, 2-36[K⁴, RYYSA¹⁹⁻²³]-PP, [cPP¹⁻⁷, NPY¹⁹⁻²³ , His³⁴]-hPP, 2-36[K⁴, RYYSA¹⁹⁻¹³]-PP, [cPP¹⁻⁷, NPY¹⁹⁻²³, Ala³¹, Aib³², Gln³⁴]-hPP, PYY, and PYY₃₋₃₆.

The terms "parenteral administration" and "administered parenterally" refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articulare, subcapsular, subarachnoid, intraspinal, and intrasternal injection and infusion.

A "patient," "subject" or "host" to be treated by the subject method may mean either a human or non-human animal.

The term "pharmaceutically acceptable carrier" refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminium hydroxide; (15) alginic acid; (16) pyrogen-free water; (17) isotonic saline; (18) Ringer's solution; (19) ethyl alcohol; (20) phosphate buffer solutions; (21) fibrin glue; (22) platelet rich plasma; and (23) other non-toxic compatible substances employed in pharmaceutical formulations.

The term "pharmaceutically-acceptable salts" refers to the relatively non-toxic, inorganic and organic acid addition salts of compounds, including, for example, those contained in compositions described herein.

The term "prophylactic" or "therapeutic" treatment refers to administration of a drug to a host. If it is administered prior to clinical manifestation of the unwanted condition (e.g., disease or other unwanted state of the host animal) then the treatment is prophylactic (it protects the host against developing the unwanted condition or lessens the extent to which the condition develop); if it is administered after manifestation of the unwanted condition, the treatment is therapeutic (it is intended to diminish, reverse, ameliorate or maintain the existing unwanted condition or side effects thereof).

The term "small molecule" refers to a compound, which has a molecular weight of less than about 5 kD, less than about 2.5 kD, less than about 1.5 kD, or less than about 0.9 kD. Small molecules may be, for example, nucleic acids, peptides, polypeptides, peptide nucleic acids, peptidomimetics, carbohydrates, lipids or other organic (carbon containing) or inorganic molecules. The term "small organic molecule" refers to a small molecule that is often identified as being an organic or medicinal compound, and does not include molecules that are exclusively nucleic acids, peptides or polypeptides.

The terms "systemic administration," "administered systemically," "peripheral administration" and "administered peripherally" refer to the administration of a subject composition, therapeutic or other material other than directly into the central nervous system, such that it enters the patient's system and, thus, is subject to metabolism and other like processes.

The term "therapeutically effective amount" refers to that amount of a modulator, drug or other molecule which is sufficient to effect treatment when administered to a subject in need of such treatment. The therapeutically effective amount will vary depending upon the subject and disease condition being treated, the weight and age of the subject, the severity of the disease condition, the manner of administration and the like, which can readily be determined by one of ordinary skill in the art.

"Transcriptional regulatory sequence" is a generic term used herein to refer to DNA sequences, such as initiation signals, enhancers, and promoters, which induce or control transcription of protein coding sequences with which they are operable linked. In preferred embodiments, transcription of one of the recombinant genes is under the control of a promoter sequence (or other transcriptional regulatory sequence) which controls the expression of the recombinant gene in a cell-type which expression is intended. It will also be understood that the recombinant gene can be under the control of transcriptional regulatory sequences which are the same or which are different from those sequences which control, transcription of the naturally-occurring forms of genes as described herein.

As used herein, the term "transfection" means the introduction of a nucleic acid, e.g., an expression vector, into a recipient cell, and is intended to include commonly used terns such as "infect" with respect to a virus or viral vector. The term "transduction" is generally used herein when the transfection with a nucleic acid is by viral delivery of the nucleic acid. The term "transformation" refers to any method for introducing foreign molecules, such as DNA, into a cell. Lipofection, DEAE-dextran-mediated transfection, microinjection, protoplast fusion, calcium phosphate precipitation, retroviral delivery, electroporation, sonoporation, laser irradiation, magnetofection, natural transformation, and biolistic transformation are just a few of the methods known to those skilled in the art which may be used (reviewed, for example, in Mehier-Humbert and Guy, Advanced Drug Delivery Reviews 57: 733-753 (2005)).

A "vector" is a self-replicating nucleic acid molecule that transfers an inserted nucleic acid molecule into and/or between host cells. The term includes vectors that function primarily for insertion of a nucleic acid molecule into a cell, replication of vectors that function primarily for the replication of nucleic acid, and expression vectors that function for transcription and/or translation of the DNA or RNA. Also included are vectors that provide more than one of the above functions. As used herein, "expression vectors" are defined as polynucleotides which, when introduced into an appropriate host cell, can be transcribed and translated into a polypeptide(s). An "expression system" usually connotes a suitable host cell comprised of an expression vector that can function to yield a desired expression product.

The term "Y receptor" refers to any of the G-protein coupled receptor for the PP-fold peptides, including receptor subtypes Y1, Y2, Y3, Y4, Y5, and Y6. Y receptors may be referred to by other names which are illustrated with respect to the Y1 receptor. For example, the Y1 receptor may also be referred to as the "neuropeptide Y Y1 receptor", "NPY Y1 receptor", "neuropeptide Y receptor subtype 1" or "NPY receptor subtype 1." Similar names are applicable to the other Y receptor subtypes. The term Y receptor is meant to refer to all such receptor subtypes as referenced by any of the possible names therefor.

The term "Y receptor agonist" refers to an agent that increases the level of a Y receptor protein and/or activates or stimulates at least one activity, such as a biological activity, of a Y receptor protein. Biological activities of Y receptor proteins include, for example, stimulation of proliferation of preadipocytes, stimulation of adipogenesis, and/or stimulation of angiogenesis.

The term "Y receptor antagonist" refers to an agent compound or molecule that decreases the level of a Y receptor protein and/or inhibits or suppresses at least one activity, such as a biological activity, of a Y receptor protein. Biological activities of Y receptor proteins include, for example, stimulation of proliferation of preadipocytes, stimulation of adipogenesis, and/or stimulation of angiogenesis.

The term "Y receptor modulator" refers to an agent (a compound or molecule) that may either up regulate (e.g., activate or stimulate), down regulate (e.g., inhibit or suppress) or otherwise change a functional property or biological activity of a Y receptor. Y receptor modulating agents may act to modulate a Y receptor protein either directly or indirectly. In certain embodiments, a Y receptor modulating agent may be a Y receptor agonist or a Y receptor antagonist.

### 2. Lipomodeling Methods

In one embodiment, the invention provides methods for lipo modeling, which can be methods that specifically increase and/or stabilize fat deposits or methods which specifically reduce fat deposits, e.g., specific increase/stabilization and/or reduction of fat depots. The methods comprise peripherally administering one or more Y receptor modulators to a subject proximally and/or into the site of a fat depot.

In another embodiment, the invention provides a method for stabilizing or increasing a fat depot or fat graft by administering a Y receptor agonist proximally (proximal to) and/or into a fat depot. Various combinations of the foregoing methods may be used for body contouring procedures, such as to reduce a fat depot or graft in one or more areas of the body and/or stabilize or augment a fat depot and/or fat graft in another area(s).

Y receptor agonists can be used, for augmenting or stabilizing fat depots and/or fat grafts, such as in facial reconstruction; breast reconstruction; liposuction revision; treatment of HIV protease inhibitor-induced facial wasting; reconstructive surgery; and/or cosmetic surgery (e.g., cosmetic augmentation of areas such as the breasts, lips, cheeks, face, and/or forehead). Cosmetic procedures also include treatments to reduce age related "wrinkles" (e.g., crow's feet, laugh lines, etc.) and filling in of acne scars. The methods may also be used in corrective procedures, for example, to fill in a depression caused by surgical procedures. In certain embodiments, the methods for augmenting or stabilizing a fat depot may be accomplished by administering a Y receptor agonist to an endogenous fat depot at a location to be augmented. Alternatively, the methods may comprise administering a Y receptor agonist in conjunction with a fat graft (close in time to implantation of the graft). Fat grafts may be implanted at any location in the body that is to be augmented. For example, fat grafts may be implanted between epidermis and muscle fascia, intramuscular, supraperiosteal, or subperiosteal tissue planes.

In various embodiments, the Y receptor modulator may be administered proximally to the fat depot and/or fat graft (e.g., at or near the depot or graft site). For example, Y receptor modulators may be administered locally to a site where lipo modeling is desired, for example, by subcutaneous injection or transdermal administration. In one embodiment, the Y receptor modulator is administered directly into one or more locations in a fat depot and/or fat graft. Y receptor modulators may be administered one or more times until a desired result is achieved and/or may be administered at the same or a different dose over time (e.g., days, weeks, months, years) the long term for maintaining a result. It may be desirable for administration to be bi-phasic or multi-phasic, for example, a higher dose may be administered until a desired result is achieved, followed by long term administration of a lower dose for purposes of maintenance. Short term administration may include, for example, administration for at least one day, two days, one week, one month, two months, or three months and long term administration may include for example, administration for at least two weeks, one month, three months, six months, one year, two years, or more. Dosing for a given time period may be carried out, for example, by single doses which are repeated at a regular intervals (e.g., injections on a daily, weekly, monthly, etc. basis) or by administration of a single extended release formulation. Appropriate dosage regimens may be determined by one of skill in the art based on the teachings herein.

### 3. Y Receptor Modulators

In various embodiments, the Y receptor modulators (e.g., agonists or antagonists) may be any type of agent that is capable of modulating (directly or indirectly) at least one biological activity of a Y receptor. Modulators include, for example, polypeptides, peptidomimetics, small molecules, nucleic acids (e.g., antisense), and/or antibodies. In certain embodiments, Y receptor antagonists may act by binding to a Y receptor or by binding to a Y receptor ligand and inhibiting the interaction between the receptor and its ligand.

In certain embodiments, a Y receptor modulator may have the ability to modulate one or more Y receptor(s) homologs, such as, for example, one or more of Y1, Y2, Y3, Y4, Y5, and/or Y6 receptors, In certain embodiments, a Y receptor modulator may not have any substantial ability to modulate other Y receptors, such as, for example, one or more of human Y1, Y3, Y4, Y5, and/or Y6, at concentrations (e.g., *in vivo*) effective for modulating the Y2 receptor. In certain embodiments, a Y receptor modulator may not have any substantial ability to modulate, for example, one or more of human Y1, Y2, Y3, Y4, and/or Y6, at concentrations (e.g., *in vivo)* effective for modulating the Y5 receptor. In certain embodiments, a Y receptor modulator may not have any substantial ability to modulate, for example, one or more of human Y1, Y3, Y4, and/or Y6, at concentrations (e.g., *in vivo*) effective for modulating a Y2/Y5 receptor heterodimer. In one embodiment, the methods disclosed herein utilize two or more Y receptor modulators that modulate two or more Y receptors including, for example, modulators of Y1, Y2, and Y5. In one embodiment, a cocktail of modulators for more than one Y receptor subtype, such as two, three four, five or all of the Y receptor subtypes, may be used. In such embodiments, the Y receptor modulators may be formulated together or administered separately.

Y receptor modulators are generally reviewed in Berglund et al., Exp. Biol. Med. 228: 217-244 (2003). Examples of Y receptor modulators are provided in the table below:

| **Receptor** | **Agonist** | | **Antagonist** | |
|---|---|---|---|---|
| | **Peptide** | **Non-peptide** | **Peptide** | **Non-peptide** |
| Y1 | [D-Arg²⁵]-NPY | | 1229U91 | SR120819A |
| | [D-His²⁶]-NPY Des-^{AA11- 18}[Cys^{7,21}, D- | | Hipskind PA, et al. J Med Chem 40:3712-3714, 1997 | BIBP3226 BIB03304 |
| | Lys⁹(Ac), D-His²⁶, Pro³⁴]-NPY | | Zarrinmayeh H, et al. J Med Chem 41:2709-2719,1998 | H394/84 LY357897 |
| | [Phe⁷, Pro³⁴]-pNPY | | Zimmerman DM, et al, Bioorg Med Chem Lett 8:473-476, 1998 | J-104870 |
| | | | Britton TC, et al. Bioorg Med Chem Lett 9:475-480, 1999 | |
| | | | Zarrinmayeh H, et al. Bioorg Med Chem Lett 9:647-652, 1999 | |
| | | | Siegel MG, et al. Tetrahedron 55:11619-11639, 1999 | |
| | | | Poindexter GS, et al. Bioorg Med Chem Lett 12:379-382, 2002 | |
| | | | Sit SY, et al. Bioorg Med Chem Lett 12:337-340, 2002 | |
| | | | Kanatani A, et al. Biochem Biophys Res Commun 266:88-91, 1999 | |
| | | | Murakami Y, et al. Bioorg Med Chem 7:1703-1714,1999 | |
| | | | Murakami Y, et al. J Med Chem 42:2621-2632, 1999 | |
| Y2 | NPY₁₃₋₃₆ | U.S. Patent | | BIIE0246 |
| | C2-NPY Cyclo S-S [Cys²⁰, Cys²⁴]pNPY U.S. Patent Publication No. 2004/0009905 | Publication No. 2005/0014742 U.S. Patent Publication No. 2005/0070534 | | Doods H, et al. Eur J Phamacol 384:R3-R5, 1999 Dumont Y, et al. Br J Pharmacol 129:1075-1088, 2000 |
| Y4 | 1229U91 [cPP¹⁻⁷, NPY¹⁹⁻²³, His³⁴]-hPP | | | |
| | 2-36[K⁴, RYYSA¹⁹⁻²³]-PP | | | |
| Y5 | [D-Trp³²]NPY [cPP¹⁻⁷, NPY¹⁹⁻²³, His³⁴]-hPP | | Rueeger H, et al. Bioorg Med Chem Lett 10:1175-1179, 2000 | CGP71683A FR233118 |
| | 2-36[K⁴, RYYSA¹⁹⁻²³]-PP | | Youngman MA, et al. J Med Chem 43:346-350, 2000 | |
| | [Ala³¹, Aib³²]-NPY [cPP¹⁻⁷, NPY¹⁹⁻²³, | | McNally JJ, et al. Bioorg Med Chem Lett 10:1641-1643,2000 | |
| | Ala³¹, Aib³², Gln³⁴]-hPP p[D-Trp³⁴]-NPY | | McNally JJ, et al. Bioorg Med Chem Lett 10:213-216,2000 | |
| | | | Kordik CP, et al. Bioorg Med Chem Lett 11:2283-2286,2001 | |
| | | | Kordik CP, et al. Bioorg Med Chem Lett 11:2287-2290,2001 | |
| | | | Norman MH, et al. J Med Chem 43:4288-4312, 2000 | |
| | | | Fotsch C, et al. J Med Chem 44:2344-2356, 2001 | |
| | | | Itani H, et al. Bioorg Med Chem Lett 12:799-802, 2002 | |
| | | | Itani H, et al. Bioorg Med Chem Lett 12:757-761, 2002 | |

A ranking of the affinities of various Y receptor ligands for the Y receptor subtypes is provided below.

| **Receptor** | **Ligand Binding Profile** | |
|---|---|---|
| Y1 | NPY ≈ PYY ≈ [Leu³¹, Pro³⁴]NPY > NPY₂₋₃₆ > NPY₃₋₃₆ ≥ PP > NPY₁₃₋₃₆ | |
| Y2 | NPY ≈ NPY₂₋₃₆ ≈ NPY₃₋₃₆ ≈ NPY₁₃₋₃₆ >> [Leu³¹, Pro³⁴]NPY | |
| Y4 | PP > PYY ≥ NPY > NPY₂₋₃₆ | |
| Y5 | NPY ≈ PYY ≈ NPY₂₋₃₆ > hPP > [D-Trp³²]NPY > NPY₁₃₋₃₆ > rPP | |
| Y6 | | 1) NPY ≈ PYY ≈ [Leu³¹, Pro³⁴]NPY >> PP (Weinberg et al., J. Biol. Chem. 271: 16435-16438 (1996)) |
| | | 2) PP > [Leu³¹, Pro³⁴]NPY > NPY ≈ PYY (Gregor et al., J. Biol Chem. 271: 27776-27781 (1996)) |

In one embodiment, the invention provides antisense molecules that may be used as Y receptor modulators. Antisense molecules useful in accordance with the methods described herein are known in the art or may be developed by one of skill in the art based on the teachings herein. For example, NPY antisense molecules have been described in U.S. Patent Publication No. 2005/0107327 and Kalra and Kalra, Methods 22: 249-54 (2000); Y2 receptor antisense molecules have been described, for example, in U.S. Patent Publication No. 2003/0162944 and Koulu et al., Ann Med. 36: 232-40 (2004); Y1 receptor antisense molecules have been described, for example, in Cheung and Cechetto, Neuroscience 98: 771-7 (2000) and Wahlestedt et al., Science 259: 528-31 (1993); and Y5 receptor antisense molecules have been described, for example, in Gong et al., Acta Pharmacol Sin. 24: 569-75 (2003).

As used herein, antisense therapy refers to administration or *in situ* generation of oligonucleotides or their derivatives which specifically hybridize or otherwise bind under cellular conditions with the cellular mRNA and/or genomic DNA encoding, for example, a Y receptor or a PP-fold polypeptide, so as to inhibit expression of that polypeptide, e.g. by inhibiting transcription and/or translation. The binding may be by conventional base pair complementarity, or, for example, in the case of binding to DNA duplexes, through specific interactions in the major groove of the double helix. In general, antisense therapy refers to the range of techniques generally employed in the art, and includes any therapy which relies on specific binding to oligonucleotide sequences.

An antisense construct (e.g., directed to a Y receptor or a PP-fold polypeptide) may be delivered, for example, as an expression plasmid which, when transcribed in the cell, produces RNA which is complementary to at least a unique portion of the mRNA which encodes a desired polypeptide. Alternatively, the antisense construct may be an oligonucleotide which is generated *ex vivo* and, when introduced into the cell, causes inhibition of expression by hybridizing with the mRNA and/or genomic sequences encoding a desired polypeptide. Such oligonucleotide probes may be modified oligonucleotides which are resistant to endogenous nucleases, e.g. exonucleases and/or endonucleases, and are therefore stable *in vivo.* Nucleic acid molecules useful as antisense oligonucleotides include phosphoramidate, phosphothioate and methylphosphonate analogs of DNA (see also U.S. Patents 5,176,996; 5,264,564; and 5,256,775). Additionally, general approaches to constructing oligomers useful in antisense therapy have been reviewed, for example, by van der Krol et al., (1988) Biotechniques 6:958-976; and Stein et al., (1988) Cancer Res 48:2659-2668.

In another embodiment, the invention provides antibodies that may be used as Y receptor modulators. Antibodies useful in accordance with the methods described herein are known in the art or may be developed by one of skill in the art based on the teachings herein. For example, antibodies directed to Y receptors or PP-fold polypeptides are commercially available from a variety of sources, including, for example, Alpha Diagnostic International, Inc. (San Antonio, TX), Abcam, Inc. (Cambridge, MA), and Research Diagnostics, Inc. (Flanders, NJ).

The term "antibody" as used herein is intended to include fragments thereof which are also specifically reactive, for example with a Y receptor or a PP-fold polypeptide. Antibodies can be fragmented using conventional techniques and the fragments screened for utility in the same manner as is suitable for whole antibodies. For example, F(ab')₂ fragments can be generated by treating antibody with pepsin. The resulting F(ab')₂ fragment can be treated to reduce disulfide bridges to produce Fab' fragments. The antibodies useful in accordance with the methods described herein are further intended to include bispecific and chimeric molecules, as well as single chain (scFv) antibodies. Also within the scope of the invention are trimeric antibodies, humanized antibodies, human antibodies, and single chain antibodies. All of these modified forms of antibodies as well as fragments of antibodies are intended to be included in the term "antibody".

Antibodies may be produced by methods known in the art. For example, a mammal such as a mouse, a hamster or rabbit may be immunized with an immunogenic form of a desired polypeptide (e.g., an antigenic fragment which is capable of eliciting an antibody response). Alternatively, immunization may involve using a nucleic acid, which *in vivo* results in expression of a polypeptide giving rise to the immunogenic response observed. Techniques for conferring immunogenicity on a protein or peptide include conjugation to carriers or other techniques well known in the art. For instance, a peptidyl portion of a desired polypeptide may be administered in the presence of adjuvant. The progress of immunization may be monitored by detection of antibody titers in plasma or serum. Standard ELISA or other immunoassays may be used with the immunogen as antigen to assess the levels of antibodies.

Following immunization, antisera reactive with a desired polypeptide may be obtained and, if desired, polyclonal antibodies isolated from the serum. To produce monoclonal antibodies, antibody producing cells (lymphocytes) may be harvested from an immunized animal and fused by standard somatic cell fusion procedures with immortalizing cells such as myeloma cell's to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with the polypeptides of the invention and the monoclonal antibodies isolated.

In other embodiments, the antibodies, or variants thereof, are modified to make them less immunogenic when administered to a subject. For example, if the subject is human, the antibody may be "humanized" such as by transplanting the complimentarity determining region(s) of the hybridoma-derived antibody into a human monoclonal antibody, for examples as described in Jones, P. et al. (1986), Nature 321, 522-525 or Tempest et al. (1991) Biotechnology 9, 266-273. Transgenic mice, or other mammals, may also be used to express humanized antibodies. Such humanization may be partial or complete.

### 4. Combination Therapies

In certain embodiments, the methods disclosed herein may utilize combination therapies comprising one or more Y receptor modulators in combination with at least one other therapeutic agent (a therapeutic agent which is not a Y receptor modulator). In one embodiment, a method for modulating a fat depot involves administering to a subject a formulation comprising at least one Y receptor modulator and at least one other therapeutic agent. In another embodiment, a method for modulating a fat depot comprises separately administering one or more Y receptor modulators and one or more therapeutic agents, e.g., wherein the Y receptor modulator(s) and therapeutic agent(s) are in separate formulations. When using separate formulations, the Y receptor modulator(s) may be administered (1) at the same as administration of the therapeutic agent(s), (2) intermittently with the therapeutic agent(s), (3) staggered relative to administration of the therapeutic agent(s), (4) prior to administration of the therapeutic agent(s), (5) subsequent to administration of the therapeutic agent(s), and (6) various combination thereof.

Various types of therapeutic agents may be administered in conjunction with a Y receptor modulator in accordance with the methods described herein. Examples of therapeutic agents include, for example, anti-inflammatory agents, immunsuppressive agents, analgesics, antimicrobial agents, anti-infective agents (such as for example, antibiotic, antiviral, and/or antifungal compound, etc.), vitamins, antioxidants, agents that promote angiogenesis, agents that promote differentiation of pre-adipocytes into adipocytes, lipolytic agents, analgesics, anti-obesity drugs or agents (e.g., an anti-obesity agent or drug which is not a Y receptor antagonist), adrenergic modulators (e.g., alpha-adrenergic or beta-adrenergic modulators), hormones, vasodilators, vasoconstrictors, lipid lowering agents, etc.

Anti-inflammatory drugs include, for example, steroidal (such as, for example, cortisol, aldosterone, prednisone, methylprednisone, triamcinolone, dexamethasone, deoxycorticosterone, and fluorocortisol) and non-steroidal anti-inflammatory drugs (such as, for example, ibuprofen, naproxen, and piroxicam). Immunosuppressive drugs include, for example, prednisone, azathioprine (Imuran), cyclosporine (Sandimmune, Neoral), rapamycin, antithymocyte globulin, daclizumab, OKT3 and ALG, mycophenolate mofetil (Cellcept) and tacrolimus (Prograf, FK506).

Antibiotics include, for example, sulfa drugs (e.g., sulfanilamide), folic acid analogs (e.g., trimethoprim), beta-lactams (e.g., penacillin, cephalosporins), aminoglycosides (e.g., stretomycin, kanamycin, neomycin, gentamycin), tetracyclines (e.g., chlorotetracycline, oxytetracycline, and doxycycline), macrolides (e.g., erythromycin, azithromycin, and clarithromycin), lincosamides (e.g., clindamycin), streptogramins (e.g., quinupristin and dalfopristin), fluoroquinolones (e.g., ciprofloxacin, levofloxacin, and moxifloxacin), polypeptides (e.g., polymixins), rifampin, mupirocin, cycloserine, aminocyclitol (e.g., spectinomycin), glycopeptides (e.g., vancomycin), and oxazolidinones (e.g., linezolid).

Antiviral agents include, for example, vidarabine, acyclovir, gancyclovir, valganciclovir, nucleoside-analog reverse transcriptaseinhibitors (e.g., ZAT, ddI, ddC, D4T, 3TC), non-nucleoside reverse transcriptase inhibitors (e.g., nevirapine, delavirdine), protease inhibitors (e.g., saquinavir, ritonavir, indinavir, nelfinavir), ribavirin, amantadine, rimantadine, relenza, tamiflu, pleconaril, and interferons.

Antifungal drugs include, for example, polyene antifungals (e.g., amphotericin and nystatin), imidazole antifungals (ketoconazole and miconazole), triazole antifungals (e.g., fluconazole and itraconazole), flucytosine, griseofulvin, and terbinafine.

Anti-obesity agents include, for example, an apolipoprotein-B secretion/microsomal triglyceride transfer protein (apo-B/MTP) inhibitor, an MCR-4 agonist, a cholecystokinin-A (CCK-A)- agonists, a monoamine reuptake inhibitor (such as sibutramine), a sympathomimetic agent, a serotoninergic agent, a dopamine agonist (such as bromocriptine), a melanocyte-stimulating hormone receptor analog, a cannabinoid receptor antagonist, a melanin concentrating hormone antagonist, leptin (the OB protein), a leptin analog, a leptin receptor agonist, a galanin antagonist, a lipase inhibitor (such as tetrahydrolipstatin, i.e. orlistat), an anorectic agent (such as a bombesin agonist), a thyromimetic agent,'dehydroepiandrosterone or an analog thereof, a glucocorticoid receptor agonist or antagonist, an orexin receptor antagonist, a urocortin binding protein antagonist, a glucagon-like peptide-1 receptor agonist, a ciliary neurotrophic factor (such as Axokine^{™} available from Regeneron Pharmaceuticals, Inc., Tarrytown, N.Y. and Procter & Gamble Company, Cincinnati, Ohio), human agouti-related protein (AGRP), orlistat, sibutramine, bromocriptine, phentermine, ephedrine, leptin, phenylpropanolamine, and pseudoephedrine.

In one embodiment, the invention provides methods for modulating fat depots comprising administering one or more Y receptor modulators in combination with one or more β adrenergic modulators. In such embodiments, the Y receptor modulators and β adrenergic modulators may be formulated separately or together. β adrenergic modulators that are useful in accordance with the methods described herein include β adrenergic agonists and β adrenergic antagonists. In a further embodiment, methods for reducing a fat depot may comprise administration of a Y receptor antagonist in combination with a β adrenergic agonist. β adrenergic agonists include, for example, bitolterol, broxaterol, clenbuterol, colterol, fenoterol, fomoterol, formoterol, isoetharine, isoproterenol (isoprenaline), isoxsuprine, mabuterol, metaproterenol, orciprenaline, picumeterol, procaterol, reproterol, rimiterol, ritodrine, salbutamol (albuterol), salmeterol, terbutaline, zinterol, and the agonists described in U.S. Pat. Nos. 4,600,710; 5,977,124; 5,776,983; and U.S. Patent Publication Nos. 2002/0128247 and 2004/0209850. In another embodiment, methods for increasing or stabilizing a fat depot or fat graft comprise administration of a Y receptor agonist in combination with a β adrenergic antagonist. Exemplary β adrenergic antagonists include, for example, acebutolol, atenolol, betaxolol, bioprolol, carteolol, labetalol, metoprolol, nadolol, penbutolol, pindolol, propanolol, levobunalol, metipranolo, timolol, and the antagonists describedin PCT Publication No. WO 98/58638.

In certain embodiments, the invention provides methods for modulating fat depots comprising administering one or more Y receptor modulators in combination with one or more α adrenergic modulators (e.g., α adrenergic agonists, and α adrenergic antagonists). Examples of α adrenergic agonists include clonidine, apraclonidine, guanfacine, guanabenz, guanfacil, rilmenidine, and moxonidine. Exemplary α adrenergic antagonists include, for example, yohimbine, regitine, prazosin, doxazosin, tamsulosin, terazosin, octopamine, phenoxybenzamine, phentolamine, hydrochlorothiazide, 5-methyl urapidil, chloroethylclonidine, bunazosin, alfuzosin, RS17053, BMY 7378, urapidil, L-765,314, nicergoline, ABT-866, cyclazosin, A322312, A 119637, fiduxosin, JTH-601, imiloxan, 2 idopropoxyidazoxan, 2-methoxyidazoxan, Rx 821002, idazoxan, piperoxan, BRL 44408, beditin, atipamezole, rawolscine, ARC 239, RS-79948, MK912, RS 79948, UIC 14304 and ethoxyidazoxan.

In certain embodiments, the invention provides methods for modulating fat depots comprising administering one or more Y receptor modulators in combination with one or more protease inhibitors, such as, for example inhibitors of dipeptidyl peptidase IV (DPPIV) and/or aminopeptidase P. Exemplary DPPIV inhibitors include, for example, LAF237 from Novartis (NVP DPP728; 1-[[[2-[(5-cyanopyridin-2-yl)amino] ethyl]amino]acetyl]-2- cyano-(S)- pyrrolidine) or MK-04301 from Merck (see e.g., Hughes et al., Biochemistry 38: 11597-603 (1999)). Other exemplary DPPIV inhibitors are described, for example, in PCT Publication Nos. WO 95/29691, WO 97/40832, WO 99/61431, WO 99/67279; WO 00/34241, WO 02/14271, WO 02/30890, WO 02/38541, WO 02/051836, WO 02/062764, WO 02/076450, WO 02/083128, and U.S. Patent Publication No. 2004/0082570. An exemplary aminopeptidase P inhibitor includes, for example, Apstatin (see e.g., Wolfrum et al., Br. J. Pharmacology 134: 370-374 (2001)).

In yet another embodiment, the methods described herein comprise administration of two or more Y receptor modulators in combination. For example, a method for reducing body fat comprising systemically administering (e.g., orally, nasally, etc.) a Y receptor agonist and peripherally administering a Y receptor antagonist (e.g., proximally to the site of a fat depot). Compositions for systemic administration of a Y receptor agonist are disclosed, for example, in U.S. Patent Publication Nos. 2004/0115135 and 2005/0009748.

In another embodiment, the invention provides methods for preventing, reducing, or treating drug induced weight gain by peripherally administering to a subject a Y receptor antagonist. The Y receptor antagonist may be administered to the subject prior to, concurrently with, and/or subsequent to administration of a weight gain inducing drug. Examples of drugs that may induce weight gain include, for example, tricyclic antidepressants, lithium, sulphonylureas, beta-adrenergic blockers, certain steroid contraceptives, corticosteroids, insulin, cyproheptadine, sodium valproate, piztifen, neuroleptics including typical neuroleptics for example phenothiazine and phenothiazine derivatives such as chlorpromazine, thioridazine, fluphenazine and trifluoperazine; butyrophenones such as haloperidol; thioxanthenes such as flupentixol and substituted benzamides such as sulpiride, atypical neuroleptics including clozapine, olanzapine, zotepine, risperidone, quetiapine and ziprasidone. In yet another embodiment, a Y receptor antagonist may be peripherally administered to treat and/or prevent weight gain associated with an anti-smoking regimen (treatment or therapy for reducing or eliminating smoking by an individual).

### 5. Grafts

In certain embodiment, the methods disclosed herein utilize fat grafts in association with a Y receptor modulator, Any type of fat tissue may be utilized, including subcutaneous depots from such areas as the chest, abdomen and buttocks, hips and waist. Visceral fat depots may also be used, such as that found above the kidneys. Fat tissue may be extracted from a subject using methods standard in the art for obtaining tissue for grafting. For example, such tissue may be surgically extracted using standard or minimally invasive surgical techniques. Minimal-invasive surgery (MIS) refers to surgical procedures using surgical and diagnostic instruments specially developed to reduce the amount of physical trauma associated with the procedure. Generally, MIS involves instruments that may be passed through natural or surgically created openings of small diameter into a body to a desired location of use so that surgical intervention is possible with substantially less stress being imposed on the patient, for example, without general anesthesia. MIS may be accomplished using visualization methods such as fiberoptic or microscopic means. Examples of MIS include, for example, arthoscopic surgery, laparoscopic surgery, endoscopic surgery, thoracic surgery, neurosurgery, bladder surgery, gastrointestinal tract surgery, etc.

In certain embodiments, tissue is removed from a subject in a size and shape suitable for implantation into a specific lesion. In other embodiments, the tissue is removed from the subject and then is altered to a desired size and shape ex vivo using standard techniques. Methods for shaping grafts are described, for example, in U.S. Patent No. 6,503,277.

In certain embodiments, the fat tissue for use in treatment of a subject may be autologous (obtained from the recipient), allogeneic (obtained from a donor subject other than the recipient), or xenogenic (obtained from a different species, e.g., a non-human donor, such as, for example, a pig). For allogeneic sources, the closest possible immunological match between donor and recipient is desired. If an autologous source is not available or warranted, donor and recipient Class I and Class II histocompatibility antigens can be analyzed to determine the closest match available. This minimizes or eliminates immune rejection and reduces the need for immunosuppressive or immunomodulatory therapy. If required, immunosuppressive or immunomodulatory therapy can be started before, during, and/or after the graft is introduced into a patient. For example, cyclosporin A, or other immunosuppressive drugs, can be administered to the recipient. Immunological tolerance may also be induced prior to transplantation by alternative methods known in the art (D. J. Watt et al. (1984) Clin. Exp. Immunol. 55: 419; D. Faustman et al., 1991, Science 252: 1701). In exemplary embodiments, autologous grafts are used. Appropriate sterile conditions may be used when extracting, handling and implanting the grafts. Such sterile techniques will be known to the skilled artisan based on the teachings herein.

In various embodiments, fat cells and/or tissue may be obtained, for example, by liposuction, lipoaspiration, and/or direct excision. In certain embodiments, fat cells and/or tissue obtained from a liposuction or lipoaspiration procedure may be used for implantation at another location in association with a Y receptor modulator. In one embodiment, fat tissue that is harvested using a more gentle procedure such that the fat tissue remains intact may be used. In yet another embodiment, cells (including adipocytes, stem cells, pre-adipocytes, etc.) isolated from fat tissue may be used in accordance with the methods disclosed herein.

In certain embodiments, the fat graft may be treated with a Y receptor agonist prior to implantation into the graft recipient. The agonist may be administered by a variety of methods. For example, treating the graft with the Y receptor agonist may involve soaking and/or coating the graft with absolution or composition comprising one or more Y receptor agonists. Coating the graft with an appropriate solution or composition may be carried out using a brush or spatula, by spraying the solution onto the surface of the graft, or by dipping the graft into the solution or composition. Treating the graft with the Y receptor agonist may also involve injecting or infusing the graft with a solution or composition comprising one or more Y receptor agonists. In certain embodiments, combinations of these or other methods may be used. When injection is used for application of the Y receptor agonists, multiple injections at different locations within the tissue may be used.

The tissue may be treated with a Y receptor agonist at various time points. For example, the tissue may be contacted with the Y receptor agonist at one or more of the following times: prior to removal of the tissue from a patient, after removal of the tissue from a patient, concurrently with implanting the tissue in the same or a different patient, and/or after implantation into the same or a different patient. In one embodiment, the tissue is contacted with one or more Y receptor agonist ex vivo, e.g., after the tissue has been removed from a patient and prior to implantation into the same or a different patient. In various embodiments, tissue may be contacted with one or more Y receptor agonists on one or more, occasions, for example, at least one, two, three, four, five, six, seven, eight, nine, ten, or more applications of the Y receptor agonist may be made to the tissue. Such applications may be made at one time (e.g., within the span of an hour) or over a period of time, for example, over at least two hours, five hours, ten hours, 24 hours, two days, three days, four days, five days, one week, two weeks, three weeks, one month, or more.

In certain embodiments, fat grafts useful in accordance with the methods described herein may comprise fat cell suspensions or fat grafts (e.g., tissue) that have been supplemented with cells. Useful cell types include, for example, cells derived from fat such as stem cells or pre-adipocytes. Cells can be unmodified or genetically engineered to produce one or more desired polypeptides, including polypeptides useful as Y receptor modulators as described herein. Methods for genetically engineering cells with retroviral vectors, polyethylene glycol, or other methods known to those skilled in the art can be used.

In specific embodiments, cells useful as fat grafts or fat graft supplements are autologous to the subject into which the fat graft will be implanted. Alternatively, cells from close relatives or other donors of the same species may be used with appropriate immunosuppression. Immunologically inert cells, such as embryonic or fetal cells, stem cells, and cells genetically engineered to avoid the need for immunosuppression can also be used. Methods and drugs for immunosuppression are known to those skilled in the art of transplantation.

In one embodiment, cells useful as fat grafts or fat graft supplements may be obtained by biopsy and may optionally be expanded in culture before application to the fat graft or recipient. Cells can be easily obtained through a biopsy anywhere in the body, for example, fat tissue can be obtained from almost any subcutaneous region. To obtain fat, the area to be biopsied can be locally anesthetized with a small amount of lidocaine injected subcutaneously.

Alternatively, a small patch of lidocaine jelly can be applied over the area to be biopsied and left in place for a period of 5 to 20 minutes, prior to obtaining biopsy specimen. The biopsy can be effortlessly obtained with the use of a biopsy needle, a rapid action needle which makes the procedure extremely simple and almost painless. With the addition of the anesthetic agent, the procedure would be entirely painless. This small biopsy core of fat can then be transferred to media consisting of phosphate buffered saline. The biopsy specimen is then transferred to the lab where the fat can be grown utilizing the explant technique, wherein the fat is divided into very small pieces which are adhered to a culture plate, and serum containing media is added.

Alternatively, the fat biopsy can be enzymatically digested with agents such as trypsin and the cells dispersed in a culture plate with any of the routinely used medias.

After cell expansion within the culture plate, the cells can be easily passaged utilizing the usual technique until an adequate number of cells are achieved.

In still other embodiments, the fat cells may be cells which naturally produce, or have been engineered to produce, a gene product of interest. Gene products of interest may include, for example, a Y receptor agonist or another polypeptide which enhances graft stability, maintenance, transformation, fat cell differentiation, etc. once implanted into a subject in need thereof. In certain embodiments, a gene product of interest may be useful to stimulate differentiation of pre-adipocytes into adipocytes in the fat graft. In other embodiments, a gene product of interest may alternatively or additionally stimulate surrounding cells of the fat graft (such as, for example, fat tissue in the graft recipient located near the site of graft implantation) to differentiate from pre-adipocytes into adipocytes. In yet another embodiment, a gene product of interest may alternatively or additionally stimulate attraction or ingrowth of pluripotent stem cells and/or progenitor cells to migrate into the tissue graft. After migration into the graft, the same or a different gene product, and/or another agent, may be used to stimulate differentiation of the stem cells and/or progenitor cells into a desired cell type, such as fat cells.

### 5. Pharmaceutical Compositions

Pharmaceutical compositions comprising Y receptor modulators for use in accordance with the methods described herein may be formulated in a conventional manner using one or more physiologically acceptable carriers or excipients. Y receptor modulators and their physiologically acceptable salts and solvates may be formulated for administration by, for example, injection, inhalation or insufflation (either through the mouth or the nose) or oral, buccal, parenteral or rectal administration. In one embodiment, a Y receptor modulators is administered locally, at the site where the target cells, e.g., fat cells or a fat graft, are present.

Y receptor modulators can be formulated for a variety of routes of administration, including systemic and topical or localized administration. Techniques and formulations generally may be found in Remmington's Pharmaceutical Sciences, Meade Publishing Co., Easton, Pa. For systemic administration, injection is preferred, including intramuscular, intravenous, intraperitoneal, and subcutaneous. For injection, the Y receptor modulators can be formulated in liquid solutions, preferably in physiologically compatible buffers such as Hank's solution or Ringer's solution. In addition, the Y receptor modulators may be formulated in solid form and redissolved or suspended immediately prior to use. Lyophilized forms are also included.

The Y receptor modulators may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

Pharmaceutical compositions (including cosmetic preparations) may comprise from about 0.00001 to 100%, such as from 0.001 to 10% or from 0.1 % to 5% by weight, of one or more Y receptor modulators described herein.

In one embodiment, a Y receptor modulator may be incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like.

Formulations may be colourless, odourless ointments, lotions, creams, microemulsions and gels.

Y receptor modulators may be incorporated into ointments, which generally are semisolid preparations which are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing. As explained in Remington's, cited above, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Exemplary Water-soluble ointment bases can be prepared from polyethylene glycols (PEGs) of varying molecular weight; again, reference may be had to Remingtion's, *supra*, for further information.

Y receptor modulators may be incorporated into lotions, which generally are preparations to be applied to the skin surface without friction, and are typically liquid or semiliquid preparations in which solid particles, including the active agent, are present in a water or alcohol base. Lotions are usually suspensions of solids, and may comprise a liquid oily emulsion of the oil-in-water type. Lotions are preferred formulations for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose, or the like. An exemplary lotion formulation for use in conjunction with the present method contains propylene glycol mixed with a hydrophilic petrolatum such as that which may be obtained under the trademark Aquaphor^{™} from Beiersdorf, Inc. (Norwalk, Conn.).

Y receptor modulators may be incorporated into creams, which generally are viscous liquid or semisolid emulsions, either oil-in-water or water-in-oil. Cream bases are water-washable, and contain an oil phase, an emulsifier and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation, as explained in Remington's, *supra*, is generally a nonionic, anionic, cationic or amphoteric surfactant.

Y receptor modulators may be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifer") is generally selected from the group of polyglycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

Y receptor modulators may be incorporated into gel formulations, which generally are semisolid systems consisting of either suspensions made up of small inorganic particles (two-phase systems) or large organic molecules distributed substantially uniformly throughout a carrier liquid (single phase gels). Single phase gels can be made, for example, by combining the active agent, a carrier liquid and a suitable gelling agent such as tragacanth (at 2 to 5%), sodium alginate (at 2-10%), gelatin (at 2-15%), methylcellulose (at 3-5%), sodium carboxymethylcellulose (at 2-5%), carbomer (at 0.3-5%) or polyvinyl alcohol (at 10-20%) together and mixing until a characteristic semisolid product is produced. Other suitable gelling agents include methylhydroxycellulose, polyoxyethylene-polyoxypropylene, hydroxyethylcellulose and gelatin. Although gels commonly employ aqueous carrier liquid, alcohols and oils can be used as the carrier liquid as well.

Various additives, known to those skilled in the art, may be included in formulations, e.g., topical formulations. Examples of additives include, but are not limited to, solubilizers, skin permeation enhancers, opacifiers, preservatives (e.g., anti-oxidants), gelling agents, buffering agents, surfactants (particularly nonionic and amphoteric surfactants), emulsifiers, emollients, thickening agents, stabilizers, humectants, colorants, fragrance, and the like. Inclusion of solubilizers and/or skin permeation enhancers is particularly preferred, along with emulsifiers, emollients and preservatives. An optimum topical formulation comprises approximately: 2 wt. % to 60 wt. %, preferably 2 wt. % to 50 wt. %, solubilizer and/or skin permeation enhancer; 2 wt. % to 50 wt. %, preferably 2 wt. % to 20 wt. %, emulsifiers; 2 wt. % to 20 wt. % emollient; and 0.01 to 0.2 wt. % preservative, with the active agent and carrier (e.g., water) making of the remainder of the formulation.

A skin permeation enhancer serves to facilitate passage of therapeutic levels of active agent to pass through a reasonably sized area of unbroken skin. Suitable enhancers are well known in the art and include, for example: lower alkanols such as methanol ethanol and 2-propanol; alkyl methyl sulfoxides such as dimethylsulfoxide (DMSO), decylmethylsulfoxide (C₁₀ MSO) and tetradecylmethyl sulfboxide; pyrrolidones such as 2-pyrrolidone, N-methyl-2-pyrrolidone and N-(-hydroxyethyl)pyrrolidone; urea; N,N-diethyl-m-toluamide; C₂-C₆ alkanediols; miscellaneous solvents such as dimethyl formamide (DMF), N,N-dimethylacetamide (DMA) and tetrahydrofurfuryl alcohol; and the 1-substituted azacycloheptan-2-ones, particularly 1-n-dodecylcyclazacycloheptan-2-one (laurocapram; available under the trademark Azone^{™} from Whitby Research Incorporated, Richmond, Va.).

Examples of solubilizers include, but are not limited to, the following: hydrophilic ethers such as diethylene glycol monoethyl ether (ethoxydiglycol, available commercially as Transcutol^{™}), and diethylene glycol monoethyl ether oleate (available commercially as Softcutol^{™}); polyethylene castor oil derivatives such as polyoxy 35 castor oil, polyoxy 40 hydrogenated castor oil, etc.; polyethylene glycol, particularly lower molecular weight polyethylene glycols such as PEG 300 and PEG 400, and polyethylene glycol derivatives such as PEG-8 caprylic/capric glycerides (available commercially as Labrasol^{™}); alkyl methyl sulfoxides such as DMSO; pyrrolidones such as 2-pyrrolidone and N-methyl-2-pyrrolidone; and DMA. Many solubilizers can also act as absorption enhancers. A single solubilizer may be incorporated into the formulation, or a mixture of solubilizers may be incorporated therein.

Suitable emulsifiers and co-emulsifiers include, without limitation, those emulsifiers and co-emulsifiers described with respect to microemulsion formulations. Emollients include, for example, propylene glycol, glycerol, isopropyl myristate, polypropylene glycol-2 (PPG-2) myristyl ether propionate, and the like.

In certain embodiments, other active agents may also be included in formulations described herein, including, for example, anti-inflammatory agents, immunsuppressive agents, analgesics, antimicrobial agents, antifungal agents, antibiotics, vitamins, antioxidants, sunblock agents, etc.

In certain topical formulations, the active agent is present in an amount in the range of approximately 0.25 wt. % to 75 wt. % of the formulation, preferably in the range of approximately 0.25 wt. % to 30 wt. % of the formulation, more preferably in the range of approximately 0.5 wt. % to 15 wt. % of the formulation, and most preferably in the range of approximately 1.0 wt. % to 10 wt. % of the formulation.

Topical skin treatment compositions can be packaged in a suitable container to suit its viscosity and intended use by the consumer. For example, a lotion or cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar. The composition may also be included in capsules such as those described in U.S. Pat. No. 5,063,507. Accordingly, also provided are closed containers containing a cosmetically acceptable composition.

Any pharmaceutically acceptable vehicle or formulation suitable for local infiltration or injection into a site to be anesthetized, that is able to provide a sustained release of Y receptor modulator may be employed. Slow release formulations known in the art include specially coated pellets, polymer formulations or matrices for surgical insertion or as sustained release microparticles, e.g., microspheres or microcapsules, for implantation, insertion or injection, wherein the slow release of the active medicament is brought about through sustained or controlled diffusion out of the matrix and/or selective breakdown of the coating of the preparation or selective breakdown of a polymer matrix. Other formulations or vehicles for sustained or immediate delivery of a Y receptor modulator to a preferred localized site in a patient include, e.g., suspensions, emulsions, liposomes and any other suitable, art known, delivery vehicle or formulation.

In on embodiment, the slow release formulation is prepared as microspheres in a size distribution range suitable for local infiltration or injection The diameter and shape of the microspheres or other particles can be manipulated to modify the release characteristics. For example, larger diameter microspheres will typically provide slower rates of release and reduced tissue penetration and smaller diameters of microspheres will produce the opposite effects, relative to microspheres of different mean diameter but of the same composition. In addition, other particle shapes, such as, for example, cylindrical shapes, can also modify release rates by virtue of the increased ratio of surface area to mass inherent to such alternative geometrical shapes, relative to a spherical shape. The diameters of injectable microspheres are in a size range, for example, from about 5 microns to about 200 microns in diameter. In an exemplary embodiment, the microspheres range in diameter from about 20 to about 120 microns.

A wide variety of biocompatible materials may be utilized to provide the controlled/sustained release of the Y receptor modulators. Any pharmaceutically acceptable biocompatible polymers known to those skilled in the art may be utilized. It is preferred that the biocompatible sustained release material degrade in-vivo over a period of less than about two years, with at least 50% of the sustained release material degrading within about one year, and more preferably six months or less. More preferably, the sustained release material will degrade significantly within one to three months, with at least 50% of the material degrading into non-toxic residues which are removed by the body, and 100% of the drug being released within a time period from about two weeks to about two months. A degradable sustained release material should preferably degrade by hydrolysis, and most preferably by surface erosion, rather than by bulk erosion, so that release is not only sustained but also provides desirable release rates. However, the pharmacokinetic release profile of these formulations may be first order, zero order, bi- or multi-phasic, to provide the desired reversible local effect over the desired time period.

In the case of polymeric materials, biocompatibility is enhanced by recrystallization of either the monomers forming the polymer and/or the polymer using standard techniques.

Suitable biocompatible polymers can be utilized as the sustained release material. The polymeric material may comprise biocompatible, biodegradable polymers such as a polylactide, a polyglycolide, a poly(lactide-co-glycolide), a polyanhydride, a polyorthoesters, polycaprolactones, polyphosphazenes, polysaccharides, proteinaceous polymers, soluble derivatives of polysaccharides, soluble derivatives of proteinaceous polymers, polypeptides, polyesters, and polyorthoesters or mixtures or blends of any of these. The polysaccharides may be poly-1,4-glucans, e.g., starch glycogen, amylose, amylopectin, and mixtures thereof. The biodegradable hydrophilic or hydrophobic polymer may be a water-soluble derivative of a poly-1,4-glucan, including hydrolyzed amylopectin, hydroxyalkyl derivatives of hydrolyzed amylopectin such as.hydroxyethyl starch (HES), hydroxyethyl amylose, dialdehyde starch, and the like. Sustained release materials which are useful in the formulations of the invention include the polyanhydrides, co-polymers of lactic acid and glycolic acid wherein the weight ratio of lactic acid to glycolic acid is no more than 4:1 (i.e., 80% or less lactic acid to 20% or more glycolic acid by weight), and polyorthoesters containing a catalyst or degradation enhancing compound, for example, containing at least 1% by weight anhydride catalyst such as maleic anhydride. Other useful polymers include protein polymers such as gelatin and fibrin and polysaccharides such as hyaluronic acid. Since polylactic acid takes at least one year to degrade in-vivo, this polymer should be utilized by itself only in circumstances where such a degradation rate is desirable or acceptable.

The polymeric material may be prepared by any method known to those skilled in the art. For example, where the polymeric material is comprised of a copolymer of lactic and glycolic acid, this copolymer may be prepared by the procedure set forth in U.S. Pat. No. 4,293,539 (Ludwig, et al.), the disclosure of which is hereby incorporated by reference in its entirety.

Various commercially available poly (lactide-co-glycolide) materials (PLGA) may be used in the preparation of the microspheres of the present invention. For example, poly(d,l-lactic-co-glycolic acid) is commercially available from Medisorb Technologies International L.P. (Cincinnati Ohio). A preferred product commercially available from Medisorb is a 50:50 poly (D,L) lactic co-glycolic acid known as MEDISORB 5050 DL. This product has a mole percent composition of 50% lactide and 50% glycolide. Other suitable commercially available products are Medisorb 65:35 DL, 75:25 DL, 85:15 DL and poly(d,l-lactic acid) (d,l-PLA). Poly(lactide-co-glycolides) are also commercially available from Boerhinger Ingelheim (Germany) under its Resomer© mark, e.g., PLGA 50:50 (Resomer RG 502), PLGA 75:25 (Resomer RG 752) and d,l-PLA (resomer RG 206), and from Birmingham Polymers (Birmingham, Ala.). These copolymers are available in a wide range of molecular weights and ratios of lactic to glycolic acid.

Pharmaceutically acceptable polyanhydrides which are useful in the present invention have a water-labile anhydride linkage. The rate of drug release can be controlled by the particular polyanhydride polymer utilized and its molecular weight. The polyanhydride polymer may be branched or linear. Examples of polymers which are useful in the present invention include homopolymers and copolymers of poly(lactic acid) and/or poly(glycolic acid), poly[bis(p-carboxyphenoxy)propane anhydride] (PCPP), poly[bis(p-carboxy)methane anhydride] (PCPM), polyanhydrides of oligomerized unsaturated aliphatic acids, polyanhydride polymers prepared from amino acids which are modified to include an additional carboxylic acid, aromatic polyanhyride compositions, and co-polymers of polyanhydrides with other substances, such as fatty acid terminated polyanhydrides, e.g., polyanhydrides polymerized from monomers of dimers and/or trimers of unsaturated fatty acids or unsaturated aliphatic acids. Polyanhydrides may be prepared in accordance with the methods set forth in U.S. Pat. No. 4,757,128, hereby incorporated by reference. For example, polyanhydrides may be synthesized by melt polycondensation of highly pure dicarboxylic acid monomers converted to the mixed anhydride by reflux in acetic anhydride, isolation and purification of the isolated prepolymers by recrystallization, and melt polymerization under low pressure (10⁻⁴ mm) with a dry ice/acetone trap at a temperature between 140°C-250°C for 10-300 minutes. High molecular weight polyanhydrides are obtained by inclusion of a catalyst which increases the rate of anhydride interchain exchange, for example, alkaline earth metal oxides such as CaO, BaO and CaCO₃. Polyorthoester polymers may be prepared, e.g., as set forth in U.S. Pat. No. 4,070,347, hereby incorporated by reference.

Proteinaceous polymers may also be used. Proteinaceous polymers and their soluble derivatives include gelation biodegradable synthetic polypeptides, elastin, alkylated collagen, alkylated elastin, and the like. Biodegradable synthetic polypeptides include poly-(N-hydroxyalkyl)-L-asparagine, poly-(N-hydroxyalkyl)-L-glutamine, copolymers of N-hydroxyalkyl-L-asparagine and N-hydroxyalkyl-L-glutamine with other amino acids. Suggested amino acids include L-alanine, L-lysine, L-phenlylalanine, L-valine, L-tyrosine, and the like.

In one embodiment, a Y receptor modulator may be administered in association with fibrin glue. Fibrin glue is made from human pooled plasma and is commercially available from a variety of sources, such as Tisseel^{™} from Baxter (Mississauga, ON, Canada).

In embodiments where the biodegradable polymer comprises a gel, one such useful polymer is a thermally gelling polymer, e.g., polyethylene oxide, polypropylene oxide (PEO-PPO) block copolymer such as Pluronic^{™} F127 from BASF Wyandotte. In such cases, the Y receptor modulator formulation may be injected via syringe as a free-flowing liquid, which gels rapidly above 30°C (e.g., when injected into a patient). The gel system then releases a steady dose of a Y receptor modulator at the site of administration.

In additional embodiments, the sustained release material can further include a bioadhesive polymer such as pectins (polygalacturonic acid), mucopolysaccharides (hyaluronic acid, mucin) or non-toxic lectins or the polymer itself may be bioadhesive, e.g., polyanhydride or polysaccharides such as chitosan.

The aforementioned biodegradable hydrophobic and hydrophilic polymers are particularly suited for the methods and compositions of the present invention by reason of their characteristically low human toxicity and virtually complete biodegradability.

In certain embodiment, the formulations described herein may be manufactured using a method that evenly disperses the Y receptor modulator throughout the formulation, such as emulsion preparation, solvent casting, spray drying or hot melt, rather than a method such as compression molding. A desired release profile can be achieved by using a mixture of polymers having different release rates and/or different percent loading of Y receptor modulator, for example, polymers releasing in one day, three days, and one week. In addition, a mixture of microspheres having one or more different Y receptor modulators, having the same or different controlled release profile, can be utilized to provide the benefits of different potencies and spectrum of activity during the course of treatment.

Methods for manufacture of microspheres are well known and include, for example, solvent evaporation, phase separation and fluidized bed coating.

In solvent evaporation procedures, the Y receptor modulator, if soluble in organic solvents, may be entrapped in the biodegradable polymer by dissolving the polymer in a volatile organic solvent, adding the Y receptor modulator to the organic phase, emulsifying the organic phase in water which contains less than 2% polyvinyl alcohol, and finally removing the solvent under vacuum to form discrete, hardened monolithic microspheres.

Phase separation microencapsulation procedures are suitable for entrapping water-soluble agents in the polymer to prepare microcapsules and microspheres. Phase separation involves coacervation of the polymer from all organic solvent by addition of a nonsolvent such as silicone oil. In one embodiment, the microspheres may be prepared by the process of Ramstack et al., 1995, in published international patent application WO 95/13799, the disclosure of which is incorporated herein in its entirety.

The biodegradable sustained release materials may be used in order to prepare sustained release implants comprising one or more Y receptor modulators. The implants may be manufactured, e.g., by compression molding, injection molding, and screw extrusion, whereby the Y receptor modulator(s) is loaded into the polymer. Implantable fibers can be manufactured, e.g., by blending the Y receptor modulator with the sustained release material and then extruding the mixture, e.g., under pressure, to thereby obtain biodegradable fibers. In certain embodiments, the Y receptor modulator may be incorporated into the implant and/or may be coated onto a surface of the implant.

In other embodiments, the sustained release material comprises an artificial lipid vesicle, or liposome. The use of liposomes as drug delivery systems is known, and comprehensive review articles on their properties and clinical applications are available; see, e.g., Barenholz and Amselem, in "Lipsome Technology" 2nd ed., G. Gregoriadis, ed., CRC Press, 1992; Lichtenberg and Barenholz, in Methods for Biochemical Analysis, 33, D. Glick, ed., 1988. A liposome is defined as a structure consisting of one or more concentric lipid bilayers separated by water or aqueous buffer compartments. These hollow structures, which have an internal aqueous compartment, can be prepared with diameters ranging from 20 nm to 10 µm. They are classified according to their final size and preparation method as: SUV, small unilamellar vesicles (0.5-50 nm); LUV, large unilamellar vesicles (100 nm); REV, reverse phase evaporation vesicles (0.5 µm); and MLV, large multilamellar vesicles (2-10 µm).

Liposomes as described herein will vary in size. In one embodiment, the liposomes have a diameter between 100 nm and 10 microns or greater. A wide variety of lipid materials may be used to form the liposomes including natural lecithins, e.g., those derived from egg and soya bean, and synthetic lecithins, the proviso being that it is preferred that the lipids are non-immunogenic and bio-degradable. Also, lipid-based materials formed in combination with polymers may be used, such as those described in U.S. Pat. No. 5,188,837 to Domb, (incorporated by reference herein).

Examples of synthetic lecithins which may be used together with their respective phase transition temperatures, are di-(tetradecanoy)phosphatidylcholine (DTPC) (23 C.), di-(hexadecanoyl)phosphatidylcholine (DHPC) (41°C) and di-(octandecanoyl) phosphatidylcholine (DOPC) (55°C). Di-(hexadecanoyl) phosphatidylcholine is preferred as the sole or major lecithin, optionally together with a minor proportion of the di-(octadecanoyl) or the di-(tetradecanoyl) compound. Other synthetic lecithins which may be used are unsaturated synthetic lecithins, for example, di-(oleyl)phosphatidyl-choline and di-(linoleyl)phosphatidylcholine. In addition to the main liposome-forming lipid or lipids, which are usually phospholipids, other lipids (e.g. in a proportion of 5-40% w/w of the total lipids) may be included, for example, cholesterol or cholesterol stearate, to modify the structure of the liposome membrane, rendering it more fluid or more rigid depending on the nature of the main liposome-forming lipid or lipids.

In one embodiment, liposomes containing a Y receptor modulator are provided in an aqueous phase where liposomes comprising the Y receptor modulator form an aqueous pharmaceutical suspension useful for administration at the desired site. This may be accomplished via injection or implantation. Liposomes may be prepared by dissolving an appropriate amount of a phospholipid or mixture or phospholipids together with any other desired lipid soluble components (e.g., cholesterol, cholesterol stearate) flowing in a suitable solvent (e.g., ethanol) and evaporating to dryness. An aqueous solution of the Y receptor modulator may then be added and mixed until a lipid film is dispersed. The resulting suspension will contain liposomes ranging in size, which may then be fractionated to remove undesirable sizes, if necessary. This fractionation may be effected by column gel chromatography, centrifugation, ultracentrifugation or by dialysis, as well known in the art.

The above method of preparation of liposomes is representative of a possible procedure only. Those skilled in the art will appreciate that there are many different methods of preparing liposomes, all of which are deemed to be encompassed by the present disclosure.

In another embodiment, a formulation comprising a plurality of microcapsules laden with a Y receptor modulator is provided. Microcapsules may be prepared, for example, by dissolving or dispersing the Y receptor modulator in an organic solvent and dissolving a wall forming material (polystyrene, alkylcelluloses, polyesters, polysaccharides, polycarbonates, poly(meth)acrylic acid ester, cellulose acetate, hydroxypropylmethylcellulose phthalate, dibutylaminohydroxypropyl ether, polyvinyl butyral, polyvinyl formal, polyvinylacetal-diethylamino acetate, 2-methyl-5-vinyl pyridine methacrylate-methacrylic acid copolymer, polypropylene, vinylchloride-vinylacetate copolymer, glycerol distearate, etc.) in the solvent; then dispersing the solvent containing the Y receptor modulator and wall forming material in a continuous-phase processing medium, and then evaporating a portion of the solvent to obtain microcapsules containing the Y receptor modulator in suspension, and finally, extracting the remainder of the solvent from the microcapsules. This procedure is described in more detail in U.S. Pat. Nos. 4,389,330 and 4,530,840, hereby incorporated by reference.

The sustained release dosage forms of the present invention preferably provide a sustained action in the localized area to be treated. For example, it would be desirable that such a formulation provides localized Y receptor modulator to the site for a period of one day, two days, three days, or longer. The formulations can therefore, of course, be modified in order to obtain such a desired result.

Microspheres and other injectable formulations described herein may be incorporated into a pharmaceutically acceptable solution (e.g., water) or suspension for injection. The final reconstituted product viscosity may be in a range suitable for the route of administration. In certain instances, the final reconstituted product viscosity may be, e.g., about 35 cps. Administration may be via the subcutaneous or intramuscular route. However, alternative routes are also contemplated, and the formulations may be applied to the localized site in any manner known to those skilled in the art, such that a localized effect is obtained. The formulations described herein can be implanted at the site to be treated.

The Y receptor modulator may be incorporated into a polymer or other sustained-release formulation in a percent loading between 6.1% and 90% or more, by weight, between 5% and 80%, or more, by weight and between 65 and 80%, or more, by weight. In an exemplary embodiment, the Y receptor modulator is loaded at about 75% by weight.

It is possible to tailor a system to deliver a specified loading and subsequent maintenance dose by manipulating the percent drug incorporated in the polymer and the shape of the matrix or formulation in addition to the form of the Y receptor modulator (e.g., free base versus salt) and the method of production. The amount of drug released per day increases proportionately with the percentage of drug incorporated into the formulation, e.g., matrix (for example, from 5 to 10 to 20%). In one embodiment, polymer matrices or other formulations with about 75% drug incorporated are utilized, although it is possible to incorporate substantially more drug, depending on the drug, the method used for making and loading the device, and the polymer.

The rate of release of Y receptor modulator or other drugs incorporated into the formulation will also depend on the solubility properties of the Y receptor modulator or drug. The greater the solubility in water, the more rapid the rate of release in tissue, all other parameters being unchanged. For example, those Y receptor modulators having pH dependent solubility will be released more rapidly at the optimum pH for those compounds. Thus, the formulation may be optimized for the desired Y receptor modulator release rate by selecting Y receptor modulators having a desired water solubility in tissue, e.g., at tissue pH.

For oral administration, the pharmaceutical compositions may take the form of, for example, tablets, lozanges, or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); preservatives (e.g., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For administration by inhalation, the pharmaceutical compositions may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin, for use in an inhaler or insufflator may be formulated containing a powder mix of the compound and a suitable powder base such as lactose or starch.

Toxicity and therapeutic efficacy of compounds can be determined by standard pharmaceutical procedures in cell cultures or experimental animals. The LD₅₀ is the dose lethal to 50% of the population). The ED₅₀ is the dose therapeutically effective in 50% of the population. The dose ratio between toxic and therapeutic effects (LD₅₀/ED₅₀) is the therapeutic index. Compounds that exhibit large therapeutic indexes are preferred. While compounds that exhibit toxic side effects may be used, care should be taken to design a delivery system that targets such compounds to the site of fat tissue in order to minimize potential damage to other cells and, thereby, reduce side effects.

The data obtained from the cell culture assays and animal studies can be used in formulating a range of dosage for use in humans. The dosage of such compounds may lie within a range of circulating concentrations that include the ED₅₀ with little or no toxicity. The dosage may vary within this range depending upon the dosage form employed and the route of administration utilized. For any compound, the therapeutically effective dose can be estimated initially from cell culture assays. A dose may be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

### EXEMPLIFICATION

The invention now being generally described, it will be more readily understood by reference to the following examples which are included merely for purposes of illustration of certain aspects and embodiments of the present invention, and are not intended to limit the invention in any way.

### EXAMPLE 1: NPY Stimulates Adipogenesis in vitro

Undifferentiated and differentiated 3T3-L1 preadipocytes and primary preadipocytes/adipocytes as well as aortic sprouts obtained from WT and mice null for Y2R, NPY and leptin (ob/ob mice) are cultured as described in the methods. Actions of NPY 1-36 or NPY 3-36 (an agonist preferring the Y2R subtype) ranging from 1x10⁻¹⁴ to 1x10⁻⁸ M, or adipokines such as leptin or VEGF, and neurotrophins, NGF or BDNF, are studied for their effects on NPY and receptor expression. Direct proliferative effects are measured by mitogenic assays and receptor mRNA levels are measured by quantitative RT-PCR (described in the methods). The size and growth of adipocytes (differentiated from pre-adipocytes or from primary cell culture) are assessed by immunocytochemistry and analysis with NIH ImageJ or Metamorph software. Media from NPY-treated and insulin-treated pre-adipocytes during differentiation are collected daily and levels of secreted leptin, NPY, adiponectin, and resistin are determined by ELISA. To confirm the involvement of specific NPY receptor (NPYR) subtypes in these different pathways, RT-PCR, quantitative real-time RT-PCR, immunohistochemistry, and specific receptor antagonists are used.

Cells (as described above) are cocultured with, or treated with, conditioned media from neuroblastoma cells (a model of sympathetic neurons) and superior cervical ganglia (SCG, see methods). Endogenous NPY and its effect on adipogenesis, and vice versa, the effect of adipokines on NPY expression are determined.

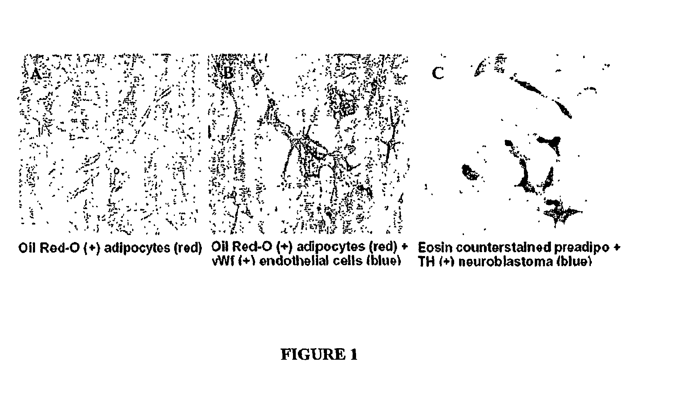

Changes in morphology of preadipocytes when cocultured with endothelial cells (HMEC's) or neuroblastoma (SKN-BE) are observed by immunocytochemistry (Fig.1). Preadipocytes appeared to flatten and differentiate to prepare for lipid filling when cocultured with endothelial cells (Fig. 1B). Endothelial cells, on the other hand, change to a more fibroblast-like appearance like the preadipocytes. Negative staining for oil red-O confirms that the preadipocytes have not yet become mature adipocytes and that there is no filling of lipids (Fig. 1B). No significant changes in the morphology of the preadipocytes are observed when they are cocultured with neuroblastoma cells (Fig. 1C). A more detailed look at the NPYR expression level by Real-time RT-PCR shows that NPY and Y1R are decreased in preadipocytes when cocultured with endothelial cells and that the Y2R is upregulated in preadipocytes when cocultured with neuroblastoma cells (Table 1).

| Preadipocytes | | Cocultured with Endothelial Cells | Cocultured with Neurons |
|---|---|---|---|
| NPY | + | ↓ | = |
| Y1R | + | ↓ | = |
| Y2R | + | = | ↑ |
| Y5R | - | - | - |
| DPPIV | + | = | = |

Table 1. Real-time RT-PCR of preadipocytes after monoculture and coculture. + indicates presence by RT-PCR, = indicates that mRNA levels are similar to preadipocytes alone, down arrow represents a 2-fold decrease in mRNA when preadipocytes are cocultured with endothelial cells, up arrow represents a 2-fold increase in mRNA when preadipocytes are cocultured with neurons

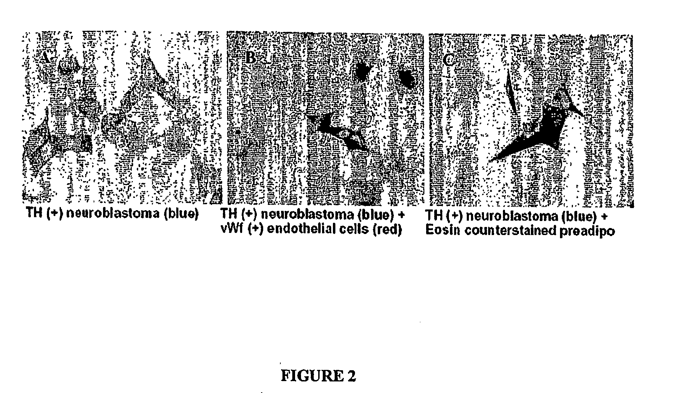

There are also changes in the morphology of neuroblastoma cells (SKN-BE) when cocultured with endothelial cells (HMEC's) or preadipocytes (3T3-L1), observed by immunocytochemistry (Fig.2). The neuroblastoma cells grow in clusters and have a more rounded appearance when cultured alone (Fig. 2A). When cocultured with endothelial cells, neuroblastoma cells tend to flatten and grow long projections to form contacts with the endothelial cells (Fig. 2B). No significant changes in the morphology of the neuroblastoma are observed when they are cocultured with preadipocytes (Fig. 2C). No changes in NPY or NPYR expression in neuroblastoma are measured by Real-time RT-PCR when they are cocultured with endothelial cells or preadipocytes (Table 2).

| Neuroblastoma | | Cocultured with Endothelial Cells | Cocultured with Preadipocytes |
|---|---|---|---|
| NPY | + | = | = |
| Y1R | - | - | - |
| Y2R | + | = | = |
| Y5R | + | = | = |
| DPPIV | - | - | - |

Table 2. Real-time RT-PCR of neuroblastoma cells after monoculture and coculture. + indicates presence by RT-PCR, = indicates that mRNA levels are similar to neuroblastoma cells alone.

Apoptosis of adipocytes or pre-adipocytes are assessed by a caspase-3/7 assay or TUNEL. Whether NPY is protective of adipocytes against apoptosis, thereby favoring adipose tissue growth, is determined.

Whether NPY's anti-lipolytic effects promote adipose tissue growth, is tested by a glycerol-release assay on basal and isoproterenol-induced free-fatty acid (FFA) release treated adipocytes.

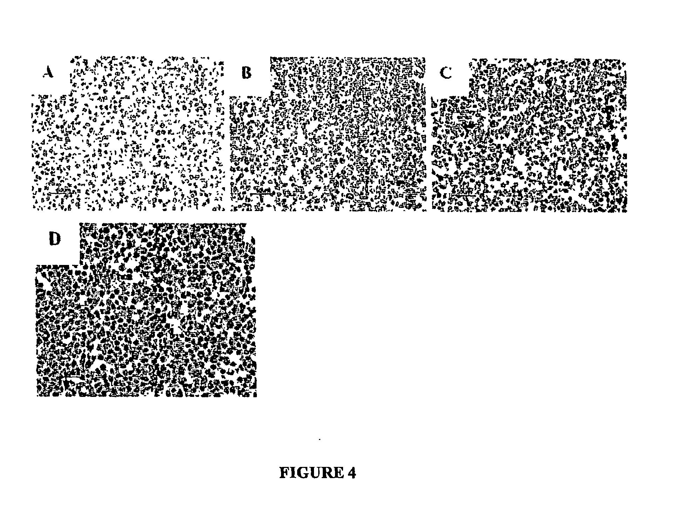

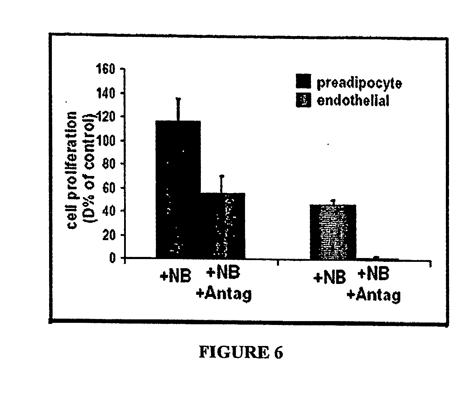

To mimic stress-released NPY *in vitro*, conditioned media from, or coculture with, neuroblastoma SKNBE cells (a model of sympathetic neurons which release 0.1pM NPY) is used to treat endothelial cells and 3T3-L1 preadipocytes. Both cell lines express all NPYRs but lack NPY (confirmed by RT-PCR and IHC, Fig. 4) and respond with proliferation which is blocked by NPYR antagonists (0.1 µM) (Fig. 6). EC are similarly treated with NB-conditioned media and also respond with proliferation, an effect that is completely blocked by a cocktail of Y1/Y2/Y5R antagonists (Fig. 6). When 3T3-L1 preadipocytes are cocultured with HMVEC, there is no significant mitogenic response between treated and untreated cells.

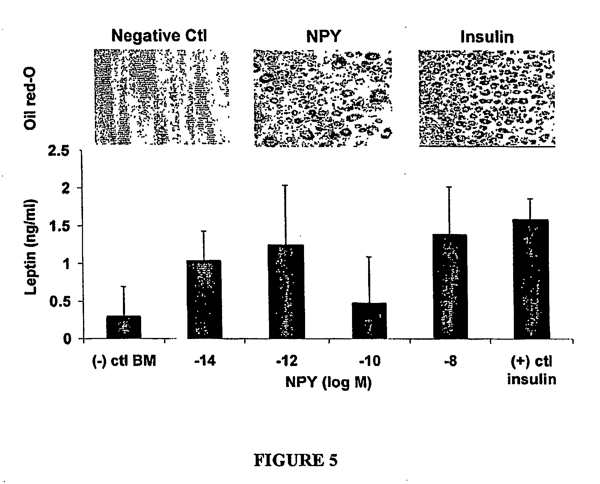

3T3-L1 preadipocytes are stimulated to differentiate to a rounded phenotype and accumulate lipids in the form of lipid droplets when treated with dexamethasone, isobutylmethylxanthine (IBMX), and insulin [54]. The 3T3-L1 cells are primed for differentiation by the incubation with IBEX and dexamethasone, and in some cells insulin, for 24 hrs, then, insulin is withdrawn and replaced with various concentrations of NPY (Fig. 5). Lipid deposition can be measured by Oil red-O staining and is markedly increased by NPY with a similar effectiveness to that of insulin (Fig. 5). In parallel to lipid deposition, NPY also increases leptin secretion in differentiated 3T3-L1 adipocytes, an indicator of the formation of functional mature adipocytes (Fig. 5). NPY is unable to stimulate adipogenesis by itself in cells which are not originally primed by insulin. This suggests cooperation between these two factors.

### EXAMPLE 2: YReceptor Agonists Stabilize Fat Grafts/Y Receptor Antagonists Reduce Fat Depots

Primary adipose endothelial cells (aECs) are obtained and cultured as described in the methods and treated with NPY 1-36 or NPY 3-36 (an agonist preferring the NPY-Y2R subtype) ranging from 1x10⁻¹⁴ to 1x10⁻⁸ M, or with adipokines such as leptin or VEGF, as well as neurotrophins, NGF or BDNF. Human aECs are used to determine upstream and downstream mediators of NPY and sympathetic angiogenesis. Murine aECs are used to study NPY's angiogenic signaling using mice deficient in proteins which may be involved in NPY-mediated angiogenesis: NPY, Y2Rs, and eNOS. Previous studies have shown that Y2R^{-/-} and eNOS^{-/-} mice lack compensatory neovascularization via NPY-mediated angiogenesis [3, 24]. These knockout mice are used to determined protein involved in NPY-mediated angiogenesis *in vivo*. Expression of NPY, its receptors (Rs) and DPPIV are determined by Real Time RT-PCR and IHC in both neuronal and endothelial cells, since some human ECs express NPY's whole autocrine system [23]. Cell proliferation and capillary tube formation on Matrigel are studied in aECs treated with NPY, NPY3-36 and synthetic Y2-specific agonist [60], with or without specific blockers: Y1 (H409/22, AstraZeneca, [32]), Y5 (L-152,804, Tocris, [32]) and Y2 R (BIIE0246, Tocris, [32]) antagonists, Y2 antisense oligonucleotide [29], and DPPIV inhibitor (P32/98, Probiodrug, [61]).

Cells (as described above) are cocultured with, or treated with, conditioned media from neuroblastoma cells (a model of sympathetic neurons) and superior cervical ganglia (SCG, see methods). Endogenous NPY and the release of other neuronal growth factors on angiogenesis are determined.

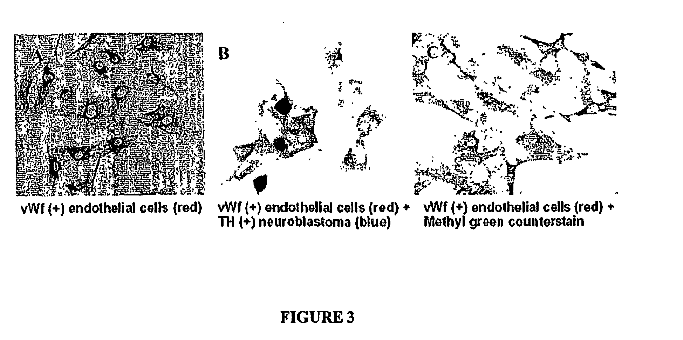

Morphology of endothelial cells is observed by immunocytochemistry. There are no significant changes in the morphology of endothelial cells when cocultured with neuroblastoma (SKN-BE) (Fig. 3B). Endothelial cells cocultured with preadipocytes vary more in appearance, with some becoming more fibroblast-like and skinny while others remain large and flat (Fig. 3C). NPYR expression level by Real-time RT-PCR shows that Y2Rs are increased by 5-fold in endothelial cells when cocultured with neuroblastoma cells (Table 3).

| Endothelial Cells | | Cocultured with Neurons | Cocultured with Preadipocytes |
|---|---|---|---|
| NPY | + | = | = |
| Y1R | + | = | = |
| Y2R | + | ↑ | = |
| Y5R | + | = | = |
| DPPIV | + | = | = |

Table 3. Real-time RT-PCR of endothelial cells after monoculture and coculture. + indicates presence by RT-PCR, = indicates that mRNA levels are similar to endothelial cells alone, up arrow represents a 5-fold increase in endothelial cell mRNA when cells are cocultured with neurons

Eight week old ob/ob mice are used to study the growth of adipose tissue when treated with growth factor NPY versus antagonist. Whether the Y2R antagonist causes regression of fat deposits is determined using an antagonist in older 3-6 month old ob/ob mice. The direct proliferative effect NPY has on the fat cells is studied by local treatment of NPY via a slow-release pellet (1 mg/pellet/14 days) into the abdominal fat pad. Fat pads (subcutaneous, abdominal, omental, subcutaneous, sternal, and interscapular) are harvested and examined to determine whether there is hyperplasia, hypertrophy, or apoptosis of the fat cells. Whether NPY-mediated angiogenesis is involved in adipose tissue growth is determined. The anti-angiogenic effects and consequences of Y2R antagonist on adipose tissue growth is determined by subcutaneous injections of Y2R antagonist BIIE0246 (0.2 ml, 1µM, daily/14 days) periabdominally into the abdominal fat pad or subcutaneous implantation of a slow-release pellet containing BIIE0246 (10 µg/14 days) followed by an evaluation of the fat deposits by MRI. These fat pads are then harvested, weighed, and stained for endothelial markers and NPYRs. Fat pads are further characterized by RT-PCR, quantitative real-time PCR, and ELISA.

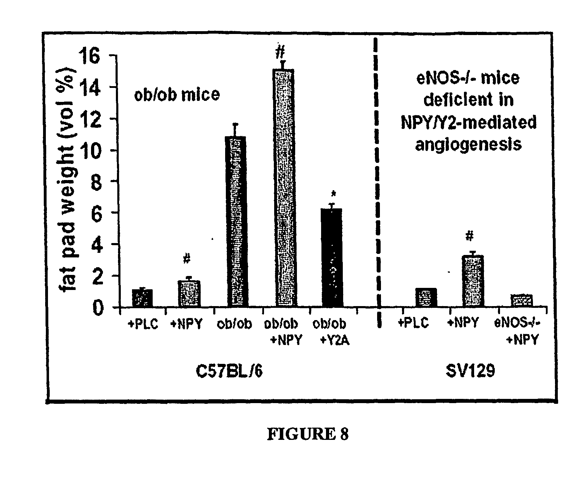

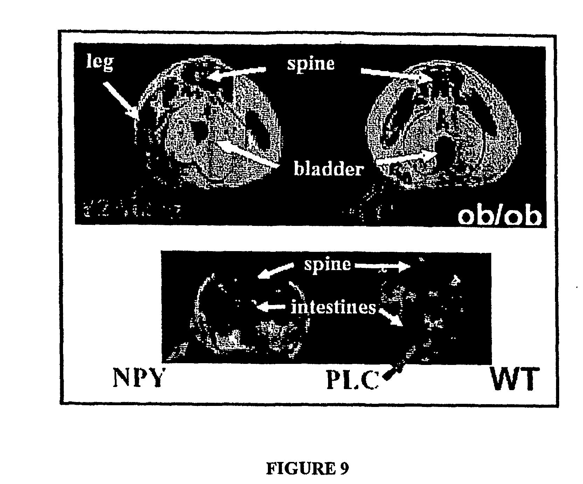

NPY slow-release pellets (1 µg/14 days) are inserted subcutaneously into the abdominal fat pad of ob/ob, C57BL/6 and SV129 WT mice. There is a significant increase in total central fat weight (FW) and FW to body weight ratio (F/B) in NPY treated versus placebo treated mice (Fig. 8). NPY pellets are also inserted into eNOS^{-/-} mice. These mice show no significant weight gain in FW or F/B (Fig. 8). This demonstrates that a component of NPY stimulation of adipose tissue growth *in vivo* operates via NO and that a component of fat pad weight gain may be attributed to NPY-mediated angiogenesis. Specific peptide Y2R antagonist, BIIE0246, is subcutaneously injected periabdominally (0.2ml, 1µM, daily/14 days) or is given in the form of a slow release pellet (10µg/14 days). Young 8-week old ob/ob mice are treated with Y2R antagonist and show decreases in both FW and F/B by 40% as compared to saline/PLC treated ob/ob mice while NPY increases visceral fat (Fig. 8). Changes in visceral fat are visualized by MRI and quantified by volumetric calculations (Fig. 9). Fat pad weight is also measured upon euthanasia of the mice (Fig. 8).

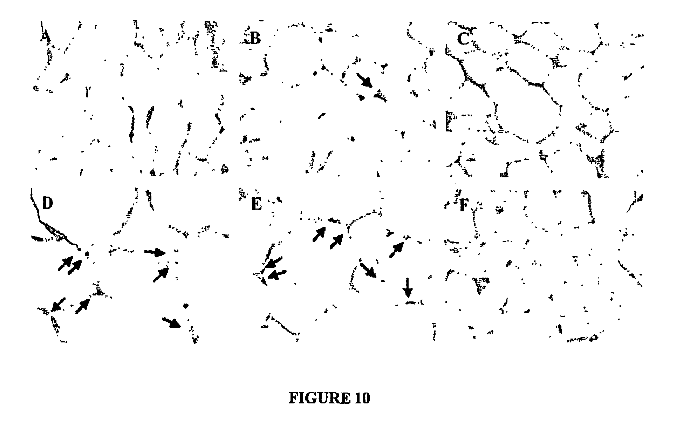

Differences in the morphology of subcutaneous abdominal fat pads are seen by immunohistochemistry (Fig. 10). No Y2R are visible on adipocytes of WT mice (Fig. 10A). There are some receptors in stressed WT mice which are, expectedly, not seen in stressed Y2R^{-/-} mice (Fig. 10B, C). The size of the adipocytes as well as the number of Y2Rs drastically increases in the fat of ob/ob mice (Fig. 10D). The Y2R-antagonist treated ob/ob mice, however, have fewer but larger adipocytes and fewer Y2Rs in general (Fig. 10E). When ob/ob mice are given NPY, their fat appears to be more like the fat of stressed WT mice (Fig. 10F). There are a greater number of smaller adipocytes as compared to placebo-treated ob/ob mice. While not wishing to be bound by theory, this could be due to NPY's proliferative effects on preadipocytes or NPY's stimulation of the differentiation of existing preadipocytes to become adipocytes. In ob/ob mice, there are fewer fat cells, but each cell is very full.

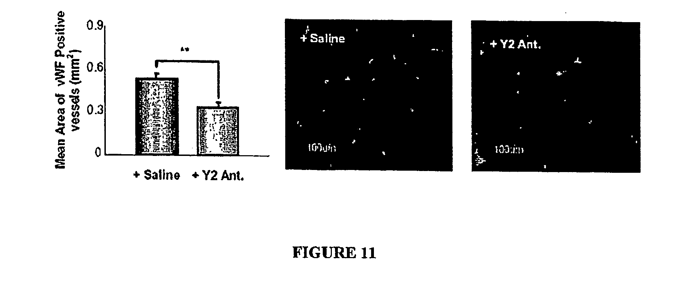

In addition to the decreased fat pad weight of Y2R-antagonist treated ob/ob mice, there is a significant decrease in capillary density stained for vonWillebrand's factor (vWf), as compared to vehicle-treated mice (Fig. 11). The mean area of vWf positive vessels is calculated in 6 random parts per section (total of 6 sections per group).

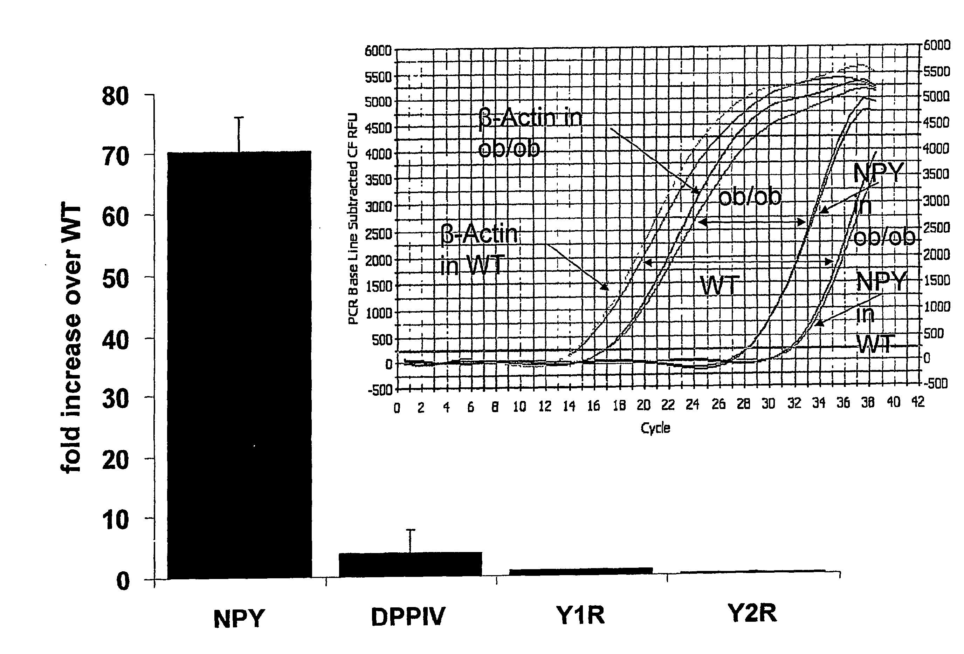

Fat pads are characterized by quantitative real-time RT-PCR (see methods) and show that obese ob/ob mice have 70-fold more NPY mRNA as compared to WT (Fig. 12), and 7-fold more DPPIV relative to WT. The presence of NPY, DPPIV, Y1R, Y2R, and Y5R are all confirmed by RT-PCR.

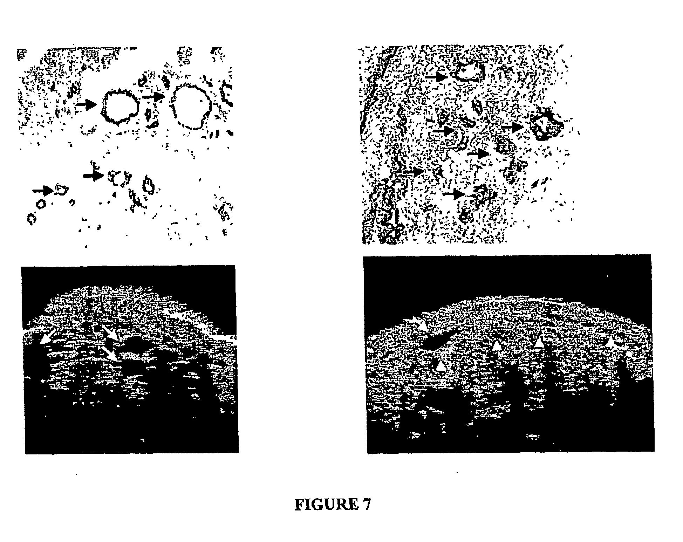

In addition to augmenting mouse fat with NPY, human fat from elective liposuction surgery is harvested by using a closed syringe system (Cell Friendly, Tulip Medical) to minimize cell trauma during harvest and aliquots of 100 µl are injected subcutaneously into a nude athymic mouse. Slow release pellets of NPY or placebo are subcutaneously implanted within cm of the fat pad, replaced 2 weeks later, and then harvested after 4 weeks of formation of fat pads. Ultrasound is used to monitor vascularization of fat pads at various time points. The time-course of expression of the NPY system and its downstream mediators is determined in relation to innervation, vascularization and fat growth (by MRI, ultrasound, IHC). Dependence of adipogenesis on angiogenesis is assessed by comparing non-specific, antiangiogenic therapy with the blockade of the NPY-Y2 angiogenic pathway. Fat pads are excised and double-stained for endothelial marker cd31 and adrenergic nerve marker tyrosine hydroxylase (Fig. 7, top). From gross observation of the fat pads and stained sections, more holes in the placebo-pellet treated fat pads are observed as compared to NPY-treated. This observation is confirmed by ultrasound (Fig. 7, bottom).

### Reference EXAMPLE 3: Role of NPY in Obesity

By administering the peptide Y2R antagonist, Applicants are able to locally/peripherally inhibit the effects of this receptor in two different models of obesity: 1) a genetically-induced model of obesity in ob/ob mice and 2) an environmentally-induced model of obesity using stress and a high-fat diet. The role of NPY/Y2R in abdominal obesity is determined.

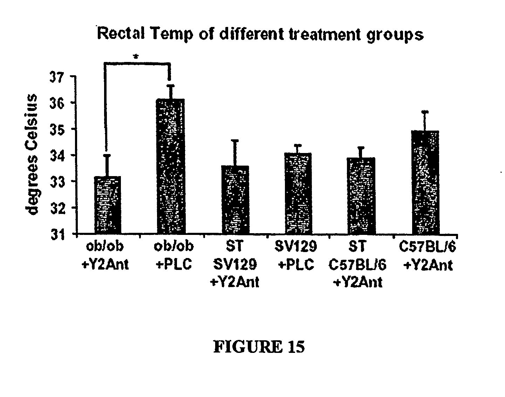

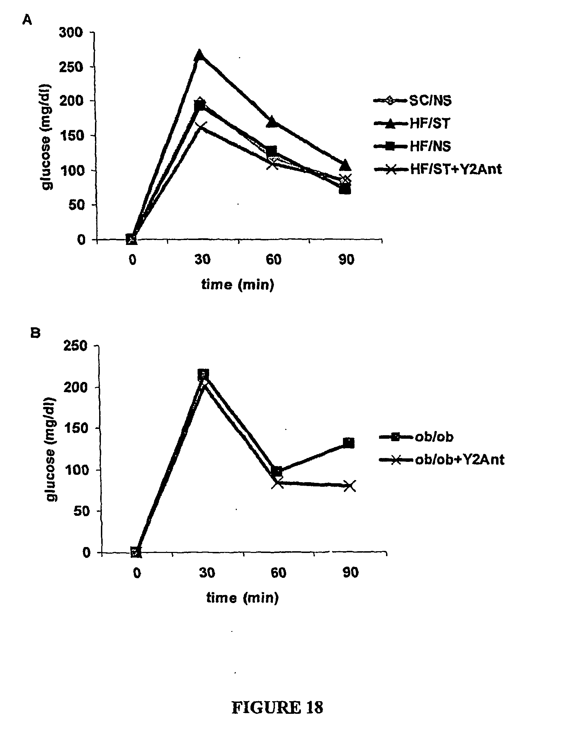

In environmentally-induced obese mice, normal WT C57BL/6 and SV129 mice are subjected to low levels of chronic stress (cold stress, 1 cm ice-water bath at the base of the cage for 1 hour [33], a technique that increases NPY and stimulates vascular growth [27]) and fed a high-fat, "comfort food" diet consisting of lard and sucrose (standard chow diet/high fat diet: fat: 12%/45%, carbohydrates: 60%/35%, and protein: 28%/20%) [44]. These mice are treated with NPY, Y2R agonists and Y2R antagonists. The growth of fat deposits is monitored by MRI and vascularization is confirmed by immunohistochemistry (IHC). ELISA, IHC, and quantitative real-time RT-PCR are used to determine how diet and stress affect NPY and Y2R levels. Core body temperature and food intake are monitored to determine changes in metabolism (Fig. 15). Glucose tolerance tests and lipid profiles of the mice are used to determine whether altering adipose mass results in a change in insulin, glucose, FFA, TG or cholesterol in the plasma (Fig.18).

The knockout mice described above are exposed to the same daily stress and high-fat diet as described above. The effects of diet and stress in the absence of NPY-signaling components are determined.

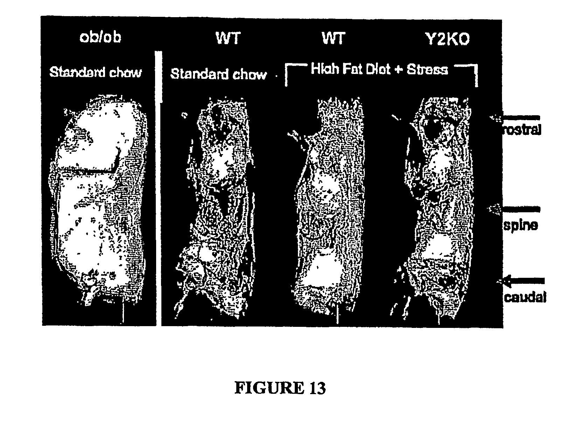

Four weeks of daily 1-hr cold exposure stress combined with a high fat diet increases visceral fat by 10±1% (fat volume fraction measurements with MRI, Fig. 13) compared to high fat fed mice, 6±2% (p<0.05 *t* test, n=6) with no effect on their food intake; similar to what the NPY pellet (1 µg/14 days) alone did (by fat-to-body weight and MRI volume fraction). This stress-amplification of visceral fat is reversed in Y2R antagonist treated mice and abolished in Y2R^{-/-} mice without affecting food consumption. The mice are euthanized after 4 weeks of treatment and fat pads are weighed to validate novel MRI calculations of fat volume fractions.

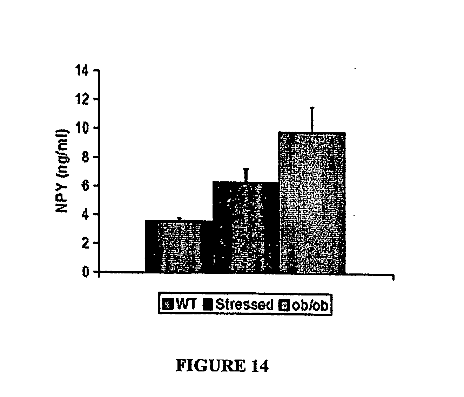

Plasma NPY levels are increased in both models of obesity: in stressed and high-fat fed C57BL/6 WT and in ob/ob, mice (Fig. 14). While not wishing to be bound by mechanism, this increase in circulating NPY may reflect a feed-forward mechanism where NPY is acting upon itself to increase angiogenesis and adipogenesis, which further increases synthesis and release of NPY from subcutaneous fat pads, resulting in dramatically increased NPY mRNA levels seen in ob/ob mice (Fig. 12).

Core body temperature measurements are monitored to examine the effects of peripheral Y2R antagonist on metabolism. There is a significant drop in core body temperature of ob/ob mice treated with Y2R-antagonist (Fig. 15), suggesting that the Y2R antagonist is not reducing adipose tissue mass via thermogenesis, which is known to be mostly mediated by β-adrenergic receptors and norepinephrine.

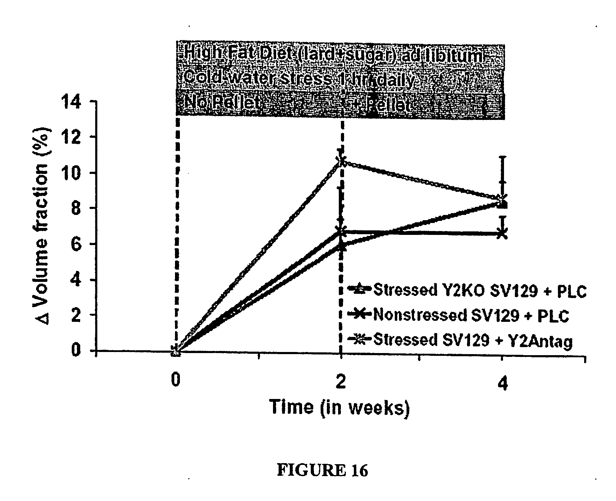

Stressed SV129 WT mice have a significant increase in visceral fat volume compared to nonstressed SV129 mice on a high-fat diet (Fig. 16). When stressed SV129 mice are given Y2R antagonist slow-release pellets, visceral fat volume fraction is reduced to a level that is comparable to nonstressed SV 129 mice given a placebo pellet (Fig. 16).

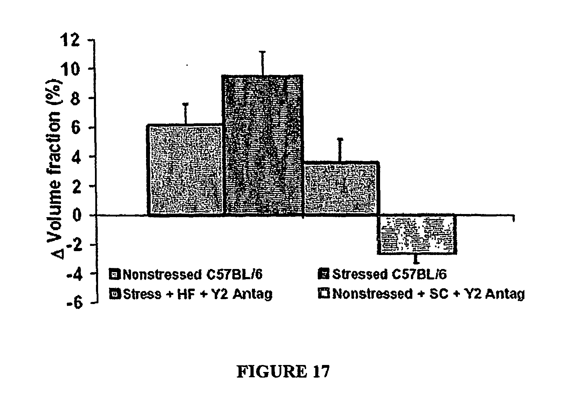

Stressed C57BL/6 WT mice have increased visceral fat volume fraction compared to nonstressed mice, this is reversed by Y2R antagonist (Fig. 17). A significant reduction in visceral fat is also seen in nonstressed WT mice treated with Y2R antagonist (Fig. 17).

In a glucose tolerance test, untreated ob/ob mice show impaired response to the glucose challenge whereas Y2R antagonist treated ob/ob mice show near normal response to the challenge (Fig. 18B). WT mice exposed to stress and given a high-fat diet also have impaired response which is resolved with Y2R antagonist treatment (Fig. 18A).

### Materials & Methods

***Isolation of aEC:*** Adipose tissue is dissected from mice or obtained from human liposuction available from the plastic surgery department at Georgetown University Hospital. The tissue fragments are washed and transported in transport medium (Medium 199, 10% FCS, 2mM glutamine, 100 IU/ml of penicillin, 100 µg/ml streptomycin and 0.25 µg/ml amphotericin B). Tissues are minced and then placed in a 25 cm² Costar Tissue flask with 15-20 mls of digestion medium (2 mg/ml collagenase, 0.5 mg/ml DNAse 1, 50 µg/ml gentamicin sulfate, 2.5 U/ml of dispase, 0.5% BSA) for 15-30 min at 37°C with intermittent shaking. After incubation, contents of the flask are transferred to a 50 mi conical tube with an equal volume of isolation medium (HBSS, 10% FCS, 2mM glutamine). The digest is pelleted by gravity in 4°C for 30 min and then the tissue fragments are centrifuged at 1500 rpm for 10 min at room temperature. Supernatants are aspirated and pellets are resuspended in 14 ml of suspension medium (HBSS, 0.35 g/L sodium bicarbonate, 0.25% BSA, 0.02% EDTA, 100 µg/ml gentamicin). The suspension is mixed thoroughly with 21 ml of iso-osmotic Perdoll and then centrifuged at 20,000g for 60 min at 4°C. The 3rd to 10th-ml fractions are transferred to a 50 ml conical tube and 30 mls of EC maintenance media (Media 199, 20% FCS, 2mM glutamine, 100 IU of penicillin, 100 µg/ml streptomycin sulfate, 0.25 µg/ml amphotericin B). This tube is centrifuged at 1,000 rpm for 10 min at room temperature and then the pellet is resuspended and plated onto fibronectin-coated culture dishes. The dishes are incubated in a humidified incubator of 95% air and 5% CO₂ maintained at 37°C. Contaminating cells are removed by flow cytometry (FACS) and FACS-sorted cells are characterized by morphology and expression vWf or cd31.

***Isolation of preadipocytes:*** The cells are isolated based on the methods as described in Hutley et al., Am J Physiol Bndocrinol Metab 281: E1037-44 (2001) and Halvorsen et al., Int J Obes Relat Metab Disord. 24(Suppl. 4): S41-4 (2000). Adipose tissue is dissected from mice or obtained from human abdominal fat available from the plastic surgery department at Georgetown University Hospital. The tissue fragments (1-5 mm) are placed in a 100 µm sterile mesh filter (Falcon 2360) and washed with Krebs-Ringer-Bicarbonate (KRB) to remove the blood. The tissues are then resuspended in KRB supplemented with 1% BSA and 1 mg/ml collagenase A (Roche) and digested at 37°C for 1 hour with intermittent shaking. The cell suspension is centrifuged at 300g for 5 min at room temperature. The stromal/vascular layer at the bottom is washed with DMEM/F12 and then incubated with iso-osmotic percoll for 5 min. Cells are plated and cultured on tissue-culture treated flasks or plates in a humidified incubator of 95% air and 5% CO₂ maintained at 37°C. Cells are characterized by morphology and some cells are plated on coverslips and stimulated to differentiate, then stained with Oil red-O to confirm whether they are preadipocytes.

***SCG cell isolation:*** Pregnant female CD IGS (Sprague-Dawley) rats are obtained from Charles River Breeding Laboratories at embryonic day 15. The rats are allowed to carry their litters to term, with delivery occurring approximately a week after arrival. 2 day old newborn rats are euthanized by decapitation. Superior cervical ganglia are dissected from each side of the neck, diced and disassociated by a combination of mechanical and enzymatic methods, according to [67, 68]. The dispersed cells are sedimented and plated on collagen coated plates in DMEM/F12 media with 10% fetal clone serum (HyClone Laboratories) and 10% NuSerum (Collaborative Biomedical). During the first week of culture, cytosine arabinofuranoside (10µM) is added to prevent proliferation of non-neuronal cells. After that, the cells are routinely cultured in serum free DMEM/F12K medium with insulin (10µg/ml), holotransferrin (20µg/ml), fatty acid free BSA (1mg/ml), 10nM 3,5,3'-triiodothyronine, metal ion mix and 100ng/ml NGF.

***Cell culture:*** Primary cells are cultured as described above and studied between passages 4 and 12. 3T3-F442A and 3T3-L1 preadipocyte cells are cultured in DMEM high glucose media with glutamine (GIBCO, Grand Island, NY), 10% calf sera (Gemini Bioproducts), d-Biotin (8µg/ml, Sigma) and Pantothenate (8µLg/ml, Sigma). SK-N-BE(2) neuroblastoma cells (ATCC) are cultured in EMEM/F12K with 10% fetal bovine sera (FBS) until 2 days postconfluent, then FCS is switched to 10% FBS (GIBCO, Grand Island, NY) and a cocktail of MDI (mixed dexamethasone and insulin) to begin differentiation. All media is supplemented with 100 units/ml penicillin, 100 µg/ml streptomycin and 2.5 mg/ml amphotericin. Aliquots of cells are preserved frozen between passages 3 and 6.

***Co-culture:*** For mitogenic assays and expression studies, co-culture is performed in a Costar Transwell system. Cells are plated on the 6-well plates and the inserts with 0.4µm Polyester membranes. After they reach 30% confluence, the inserts are placed into plates with other cells. Co-culture of neuroblastomas/SCG neurons with aECs are carried out in EC media and with preadipocytes - in preadipocyte growth media. As desired, the media is additionally supplemented with 100ng/ml NGF. After the desired period of time, the cells growing in the plates are harvested for RNA isolation. For mitogenic assay, cells are first growth-arrested in serum-free media for 24h, then the inserts placed into the plates and cells treated with desired factors in 0.25% FBS. After 48h, the cells are counted with a coulter counter. For immunostaining, aECs and pre-adipocytes are plated on cover slips in their growth media and neuroblastoma or SCG cells are added after two days of culture. The cells are treated with desired factors and fixed with 4% paraformaldehyde. In both coculture models, media is collected for ELISA.

***Mitogenic assays:*** 3T3-L1 pre-adipocytes are allowed to grow to 30% confluency before serum-starving the cells. After 24 hrs of serum-free DMEM, cells are treated for 48 hrs with either a NPY agonist or Y2R antagonist BIIE0246 (Boehringer Ingelheim Pharma; Biberach, Germany). NPY agonists include: 1) NPY 1-36 (1x10⁻¹⁴ to 1x10⁻⁸ M), 2) conditioned media from SKN-BE cells or HMVEC, or 3) co-culture with either SKN-BE or HMVEC (method described above). Proliferation is determined by cell mounting using a coulter counter (Beckman Coulter, Miami, FL). DNA Synthesis ([³H] thymidine) assays are also used to evaluate the mitogenic properties of the pre-adipocytes and their response to NPY 1-36 agonist at concentrations ranging from 1x10⁻¹⁴ to 1x10⁻⁶M. Cells plated onto 96-well dishes are growth-arrested in serum-free media for' 24 h and then treated with the desired factors for 24 h in the culture media supplemented with 0.25% FBS; 4h after treatment 0.5µCi [³H] thymidine is added to each well. After 24 h, cells are harvested in a 96-well harvester (Tomtec) and counted in a Betaplate Liquid Scintillation Counter (Wallac).

***Flow cytometry:*** Fluorescence Activated Cell Sorter (FACS) is used to measure cell size and growth of adipocytes (Becton Dickinson FACStar Plus dual laser system and a FACSort system).

***Capillary tube formation:*** aECs are incubated on Matrigel-coated 24-well plates (4x10⁴ cells/well) in full serum culture media containing the desired treatment at 37°C. After 18h, the cells are fixed and stained and the area of the tube network is quantified using an NIH image system.

***Aortic ring assay:*** Aortic rings are prepared from the thoracic aorta from mice as previously described [23]. The rings are embedded in Matrigel in Nunc 8-well chamber slides (Nalge Nunc International) and grown in Medium 199 containing 10% serum supplemented with Endothelial cell culture supplement (Sigma). Once sprouts begin to appear, the medium is changed to Medium 199 alone and treatments added for 2-3 days. Then, sprouts are photographed, fixed and stained for image analysis and quantification using NIH image.

***TUNEL:*** TUNEL reaction is performed using In Situ Cell Detection Kit (Roche Diagnostic). The cells are counterstained with DAPI (Molecular Probes) and counted using Metamorph software (Universal Imaging Corporation). Tissues are stained as above and signal converted to visible light using AP-converter. The density of TUNEL positive cells is measured using NIH Scion Image software (Scion Corp.).

***Caspase 3*/*7 activity assay:*** Cells are plated onto 96 well plates, cultured for 2 days and then treated with desired factors for 24h. The caspase 3/7 activity is measured using Apo-ONE™ Homogeneous Caspase-3/7 Assay kit (Promega, Madison, WI).

***DPPIV activity assay:*** Proteolytic activity of DPPIV is measured calorimetrically, using p-nitroanilide (pNA)-conjugated Gly-Pro dipeptide substrate (Sigma), according to [69]. The reaction is performed in 96-well plates and the amount of product measured by absorbency at 405nm.

***Glycerol-Release assay:*** Lipolysis is measured using the glycerol release assay. Cultures (day 8 of coculture/day 11 adipocytes) are incubated in lipolysis medium (3% fatty acid-poor serum albumin in Krebs-Ringer phosphate, pH 7.4) plus the indicated treatment at 37°C for 90 min. The medium is frozen at 20°C until the enzyme-coupled glycerol assay (Sigma Diagnostics Kit 337) is performed.

***RT-PCR and Real-time PCR of NPY, NPYRs and DPPIV mRNAs:*** RNA is isolated using Tri Reagent (Sigma) and cDNA synthesized by Super Script II Reverse Transcriptase (Invitrogen Life Technologies, Carlsbad, CA) using random hexamers (Perkin Elmer, Foster City, CA). The cDNA is amplified by PCR for 40 cycles (denaturing at 94°C for 1 min, annealing at 60°C for 1 min 20 sec, and extension at 72°C for 1 min) using Taq DNA polymerase (Promega). The reactions are carried out in the presence of primers specific for the sequences of the investigated genes: NPY, Y1, Y2, Y4, Y5, and DPPIV (as described in [23]). Primers for 18s rRNA are added to each reaction as an internal control. PCR products are analyzed by DNA electrophoresis on a 2.5% agarose gel and visualized by ethidium bromide staining. Real-time RT-PCR is performed using the iCycler iQ Real-time PCR detection system (Biorad, Hercules, CA). Samples are run in duplicate, each consisting of 2 µl of cDNA (obtained from total RNA as previously described), and are amplified in 20 µl of 1X TaqMan^{®}Universal PCR Master Mix (Applied Biosystems, Foster City, CA) containing primers (300 nM each) and FAM™-labeled TaqMan^{®} MGB probe (200 nM). Primers and probes (NPY: 5'-CCAGAAC TCGGCTTGAAGACCCTGC-3'; DPPIV: 5'-TGAAGCAGCCAGACA ATTTTCAAAA-3'; Y1: 5'-TCCAAAGAGGATTGTTCAGTTCAAG-3'; Y2: 5'-AGGTCC AGGTCAGTTGTAGACTCTT-3'; Y5: 5'-AAGAAAGGATTGATTCAAGAAAGAC-3';β-actin: 5'-ATGCCCCCCCCATGCCATCCTGCGT-3') are designed and synthesized by Applied Biosystems (Assay-on-Demand). Primers (see above) and probes are amplified by PCR for 40 cycles (denaturing at 95°C for 45 sec, annealing at 58°C for 45 sec, and extension at 60°C for 1 min) using TaqMan^{®} Universal PCR Master Mix. The results are analyzed using iCycler iQ software, version 3.0, provided by BioRad and expression levels calculated by the comparative CT method using β-action as an endogenous reference gene, according to the Applied Biosystems' ABI PRISM 7700 User Bulletin #2.

***ELISA:*** NPY ELISA system are purchased from Penninsula Laboratories, bFGF, VEGF and mouse leptin ELISA kits from R&D systems, mouse adiponectin ELISA kit from Linco Research and catecholamine detection system - 3CAT EIA from Diagnostic Systems Laboratories. The assays are performed according to the manufacturers' procedures.

***In vivo treatment of murine model of obesity with antagonists:*** 6-week-old mice from four murine strains: C57BL/6, ob/ob, SV129, and eNOS^{-/-} are used. The mice are separated into treatment groups to compare the effects of diet, stress, NPY and specific receptor antagonists. The mice are weighed every other day during the course of the treatment. Control groups for the transgenic mice are matched by background strain, age, and gender. WT C57BL/6 mice (a background strain predisposed to weight gain [64]) is fed a comfort food diet" consisting of lard and sugar and subjected to cold stress (1 hour in a cage with 1 cm of ice and water at the base of the cage [33]). NPY is delivered locally by the implantation of a NPY slow-release pellet (1 mg/pellet/14 days) into the abdominal fat pad. NPY receptor antagonists or antisense ODN is delivered by rotating daily subcutaneous injections (0.2 ml, 1µM, daily/14 days) in the abdominal region. 20-mer phosphorothioate oligonucleotide antisense, targeted forward from rat Y2-gene transcription initiation codon (5- CCTCTGCACCTAATGGGCCC-3') (Molecular Research Laboratories, Herndon, VA) is used. The scrambled antisense sequence contains some modified deoxynucleotides in a random order (5'-CCATGGTAATCCGCCGCTCC-3').

***Tissue sample collection and processing:*** After treatment, fat pads are harvested from: 1) subcutaneous abdominal, 2) intra-abdominal (omental), 3) inguinal, 4) interscapular. The total central fat (abdominal region only) weight (FW) and FW to percent of body weight (F/B) is measured. The adipose tissue is processed for paraffin embedding and section, 0.4 µm thick, are collected on glass slides for immunohistochemistry. 50 mg of tissue is used for quantitative real-time RT-PCR.

***Tissue processing, immunohistochemistry and image analysis:*** Samples are fixed in Histochoice (Amresco, Solon, OH) or 10% buffered formalin (Fisher) and paraffin-embedded. Slides with 0.4 µm thick paraffin sections are prepared using a microtome and stored at 4°C. Morphology is studied using sections stained with hematoxylin-eosin and receptors are characterized by immnostaining according to the manufacturers' protocols using the following Antibodies (Ab): rabbit polyclonal anti-NPY and mouse monoclonal anti-TH Ab (Immunostar), NPY (rabbit antiserum, Peninsula), Y1, Y5 (rabbit polyclonal), DPPIV (monoclonal antibody E26), rat antimouse CD31 and mouse anti-human CD31 Ab (BD Pharniingen), BS-1 Lectin (endothelial marker purchased from Vector Labs), mouse monoclonal Ab against microtubule-associated protein 2, MAP2 (Sigma), rabbit polyclonal antisera against GLUT4 and aP2 [70, 71], rabbit polyclonal Ab against human and mouse Pref and AD-3 (Alpha Diagnostic International, Inc) and rabbit polyclonal anti-mouse Ki67 Ab (DAKO). Subsequently, either staining with fluorescein-conjugated secondary antibodies are used or visualization with a streptavidin-biotin complex technique. The sections are collected and evaluated using a Nikon eclipse E 600 photomicroscope (Nikon, Inc., Melville, NY) equipped with epifluorescence.

***3T3 murine adipocyte implantation:*** 3T3-F442A preadipocytes are suspended in calf serum. BALBycAnNCrlnuBR athymic mice (Charles River Laboratories) are anesthetized by inhalation of isoflurane 1-3% in oxygen. Then, slow release pellets containing desired treatments are inserted into the abdominal area and preadipocytes injected s.c. (3x10⁷ cells per site) in their close proximity. The fat pad volume is measured periodically using MRI, whereas vascular density and caliber are measured by ultrasonography. After the desired period of time, the mice are sacrificed, fat pads harvested, measured and weighed and the samples frozen or fixed for immunohistochemical analysis.

***Human fat implantation:*** The processed fat for injection is loaded into syringes. The nude mice are anesthetized and slow release pellets inserted as described above. A small nick is made with a pair of sterile scissors in the close proximity of the pellets and 100 µl of fat is implanted using an 18 gauge Coleman infiltration cannula (Byron Medical) attached to the syringe. If necessary, the skin wound is closed with medical cyanoacrylate and the mouse is allowed to recover. The fat pad growth is monitored and the sample harvested as described above.

***Local denervation:*** In both of the implantation models, local denervation of the growing fat pads are performed by injection of 10% phenol [72] or 100 mg/kg, s.c. guanethidine [73], as previously described.

***Ultrasonography:*** The mice are anesthetized by inhalation of isoflurane 1-3% in oxygen, hair removed with depilatory cream and a sterile water based ultrasonic gel is applied to the area (Aquasonic gel - Visual Sonics). The mouse is placed in the Visual Sonics mouse holder containing a thermostatically controlled heating pad to maintain mouse body temperature. The imaging is performed with a small animal ultrasound system, Visual Sonics Vivo 660 (55 MHz).

***Magnetic Resonance Imaging (MRI):*** A Brukker 7-Tesla small-animal magnetic resonance imager coil is used to visualize and noninvasively quantitate various fat depots. A 3-dimensional (3D) T2-weighted (T2W) imaging protocol optimized for high contrast fat-imaging is implemented. This 3-D T2W RARE (Rapid Acquisiton with Relaxation Enhancement) imaging sequence TE 5.9, TR 200, Rare Factor 8, Matrix 256X128X128, 7X3X3cm - 9X3X5cm (Cranial-Caudal x AP x LR) produces a reconstructed image that shows fat as the brightest signal while signals from other tissues are relatively suppressed. Quantification of the total body fat as well as separate specific fat depots is calculated using thresholding and voxel count plugins from NIH image J software. VolumeJ is used to render, in a 3D image, the various fat depots. The animal management system that is used in conjunction with the MRI is equipped to take core, skin, ambient and water blanket temperature measurements that are monitored during the imaging. The water blanket is used to regulate core temperature of the rodent during anesthesia.

***Oil Red O staining:*** Oil Red O solution (0.36% Oil Red O solution in 60% isopropanol) is used to stain lipid droplets in mature adipocytes and paraffin embedded tissues (Chemicon Adipogenesis Assay kit).

***Hypoxia measurement:*** Hypoxyprobe™-1 Kit from Chemicon International identifies hypoxic tissues that have less than 10 mm Hg PO₂. Hypoxy-1 probe is administered I.P. and tissues of interest are isolated and analyzed immunohistochemically for localization of hypoxic cells that have formed a protein adduct with Hypoxy-1.

***Lipid analysis:*** Plasma free-fatty acids and triglyceride are quantified using a diagnostic kit from Wako Chemicals.

***Glucose tolerance test:*** After an overnight 17 h fast, unanesthetized mice are injected i.p. with a dose of 1.5 g of 50% glucose solution per kg of body weight. Blood samples are obtained from the tail vein 30 min prior to the glucose challenge and then again at 0, 30,60,90 min after the glucose challenge. Blood glucose concentrations are measured with an Elite XL (Bayer) portable glucose meter [74].

***Blood pressure:*** Animals are allowed to acclimate to the laboratory for 1 hour. Blood pressure is measured using a standard tail-cuff blood pressure plythesmography for 10 trials and averaged over the last 8 measurements.

***Stress:*** Cold-stress is achieved by placing mice in 1.0 cm ice water for 1 hour daily for 14 days.

***High fat diet:*** Mice are given either a Standard Chow (SC) diet: 28% protein, 60% carbohydrate, 12% fat (4 kcal/gm) or a High Fat (HF) diet: 20% protein, 35% carbohydrate, 45% fat (4.7 kcal/gm) from Research Diets for 14-28 days. Food consumption is measured daily from the various treatment groups and pairfeeding ensures that changes seen in weight and visceral fat are due to the peripheral effects of the treatment.

***Materials:*** NPY and its derivatives, are purchased from Penninsula Laboratories, anti-bFGF Ab, VEGFR2/Fc chimera, VEGF, bFGF and NGF from R & D systems. NPY R antagonists: Y1 - H409/22 acetate (Astra Zeneca), Y2 - BIIE0246TF (Boehringer Ingelheim Pharma), Y5 - L-152,804 (Tocris). Y2R agonist - [Ahy⁵⁻²⁴] (University of Leipzig, Germany) and DPPIV inhibitor, P32/98, from Probiodrug, Germany. Slow release pellets containing desired factors are obtained from Innovative Research of America.

### References

1. Rupnick, M.A., et al., Adipose tissue mass can be regulated through the vasculature. Proc Natl Acad Sci U S A, 2002. 99(16): p. 10730-5.
2. Turtzo, L.C., R. Marx, and M.D. Lane, Cross-talk between sympathetic neurons and adipocytes in coculture. Proc Natl Acad Sci U S A, 2001. 98(22): p. 12385- 90.
3. Lee, E.W., et al., Neuropeptide Y induces ischemic angiogenesis and restores function of ischemic skeletal muscles. J Clin Invest, 2003. 111(12): p. 1853-62.
4. Blumenthal, J.B., et al., Novel neuropeptide Y1 arzd Y5 receptor gene variants: associations with serum triglyceride and high-density lipoprotein cholesterol levels. Clin Genet, 2002. 62(3): p. 196-202.
5. Clark, J.T., et al., Neuropeptide Y and human pancreatic polypeptide stimulate feeding behavior in rats. Endocrinology, 1984. 115(1): p. 427-9.
6. Tatemoto, K., Isolation and characterization of peptide YY(PYY), a candidate gut hormone that inhibits pancreatic exocrine secretion. Proc Natl Acad Sci U S A, 1982. 79(8): p. 2514-8.
7. Colmers, W.F. and C. Wahlestedt, The Biology of neuropeptide Y and related peptides. Contemporary neuroscience. 1993, Totowa, N.J.: Humana Press. xvi, 564 p.
8. Clark, J.T., P.S. Kalra, and S.P. Kalra, Neuropeptide Y stimulates feeding but inhibits sexual behavior in rats. Endocrinology, 1985. 117(6): p. 2435-42.
9. Tomaszuk, A., C. Simpson, and G. Williams, Neuropeptide Y, the hypothalamus and the regulation of energy homeostasis. Horm Res, 1996. 46(2): p. 53-8.
10. Inui, A., Neuropeptide Y feeding receptors: are multiple subtypes involved? Trends Pharmacol Sci, 1999. 20(2): p. 43-6.
11. Billington, C.J., et al., Effect intracerebroventricular injection of neuropeptide Y on energy metabolism. Am J Physiol, 1991. 260(2 Pt 2): p. R321-7.
12. Stanley, B.G. and S.F. Leibowitz, Neuropeptide Y: stimulation of feeding and drinking by injection into the paraventricular nucleus. Life Sci, 1984. 35(26): p. 2635-42.
13. Gehlert, D.R., Role of hypothalamic neuropeptide Y in feeding and obesity. Neuropeptides, 1999. 33(5): p. 329-38.
14. Kalra, S.P., et al., Interacting appetite-regulating pathways in the hypothalamic regulation of body weight. Endocr Rev, 1999. 20(1): p. 68-100.
15. Rahmouni, K. and W.G. Haynes, Leptin signaling pathways in the central nervous system: interactions between neuropeptide Y and melanocortins. Bioessays, 2001. 23(12): p. 1095-9.
16. Cheng, X., et al., Regulation of expression of neuropeptide Y Y1 and Y2 receptors in the arcuate nucleus of fasted rats. Brain Res, 1998. 792(1): p. 89-96.
17. Batterham, R.L. and S.R. Bloom, The gut hormone peptide YY regulates appetite. Ann N Y Acad Sci, 2003. 994: p. 162-8.
18. Naveilhan, P., et al., Attenuation of hypercholesterolemia and hyperglycemia in ob/ob mice by NPY Y2 receptor ablation. Peptides, 2002. 23(6): p. 1087-91.
19. Sainsbury, A., et al., Y2 receptor deletion attenuates the type 2 diabetic syndrome of ob/ob mice. Diabetes, 2002. 51(12): p. 3420-7.
20. Sainsbury, A., et al., Important role of hypothalamic Y2 receptors in body weight regulation revealed in conditional knockout mice. Proc Natl Acad Sci U S A, 2002. 99(13): p. 8938-43.
21. Williams, R.H. and J.D. Wilson, Williams textbook of endocrinology. 9th ed. 1998, Philadelphia: Saunders. xix, 1819 p.
22. Karvonen, M.K., et al., Leucine7 to proline7 polymorphism in the preproneuropeptide Y is associated with the progression of carotid atherosclerosis, blood pressure and serum lipids in Fininsh men. Atherosclerosis, 2001. 159(1): p. 145-51.
23. Zukowska-Grojec, Z., et al., Neuropeptide Y: a novel angiogenic factor from the sympathetic nerves and endothelium. Circ Res, 1998. 83(2): p. 187-95.
24. Lee, E.W., et al., Impaired angiogenesis in neuropeptide Y (NPY)-Y2 receptor knockout mice. Peptides, 2003. 24(1): p. 99-106.
25. Kitlinska, J., et al., Dual role of dipeptidyl peptidase IV (DPP IV) in angiogenesis and vascular remodeling. Adv Exp Med Biol, 2003. 524: p. 215-22.
26. Ekstrand, A.J., et al., Deletion of neuropeptide Y (NPY) 2 receptor in mice results in blockage of NPY-induced angiogenesis and delayed wound healing. Proc Natl Acad Sci U S A, 2003. 100(10): p. 6033-8.
27. Li, L., et al., Neuropeptide Y-induced acceleration of postangioplasty occlusion of rat carotid artery. Arterioscler Thromb Vasc Biol, 2003. 23(7): p. 1204-10.
28. Kitlinska J, A.K., Kuo L, Pons J, Yu M, Li L, Tilan J, Zukowska Z, Toretsky J., Differential effects of neuropeptide Y on growth and vascularization of neural crest-derived tumors. Cancer Res, 2004.
29. Koulu, M., et al., Neuropeptide Y and Y2-receptor are involved in developntent of diabetic retinopathy and retinal neovascularization. Ann Med, 2004. 36(3): p. 232-40.
30. Morgan, C.A., 3rd, et al., Neuropeptide-Y, cortisol, and subjective distress in humans exposed to acute stress: replication and extension of previous report. Biol Psychiatry, 2002. 52(2): p. 136-42.
31. Hansel, D.E., B.A. Eipper, and G.V. Ronnett, Neuropeptide Y functions as a neuroproliferative factor. Nature, 2001: 410(6831): p. 940-4.
32. Pons, J., et al., Mitogenic actions of neuropeptide Y in vascular smooth muscle cells: synergetic interactions with the beta-adrenergic system. Can J Physiol Pharmacol, 2003. 81(2): p. 177-85.
33. Zukowska-Grojec, Z., et al., Stress-induced mesenteric vasoconstriction in rats is mediated by neuropeptide Y Y1 receptors. Am J Physiol, 1996. 270(2 Pt 2): p. H796-800.
34. Zukowska-Grojec, Z., et al., Cardiovascular, neuropeptide Y, and adrenergic responses in stress are sexually differentiated. Physiol Behav, 1991. 49(4): p. 771-7.
35. Thorsell, A., et al., Behavioral and endocrine adaptation, and up-regulation of NPY expression in rat amygdala following repeated restraint stress. Neuroreport, 1999. 10(14): p. 3003-7.
36. Eliot, R.S., Stress and cardiovascular disease: mechanisms and measurement. Ann Clin Res, 1987. 19(2): p. 88-95.
37. Engler, M.B. and M.M. Engler, Assessment of the cardiovascular effects of stress. J Cardiovasc Nurs, 1995. 10(1): p. 51-63.
38. Park, H.S., et al., Obesity, abdominal obesity, and clustering of cardiovascular risk factors in South Korea. Asia Pac J Clin Nutr, 2003. 12(4): p. 411-8.
39. Okosun, I.S., et al., Abdominal obesity defined as a larger than expected waist girth is associated with racial/ethnic differences in risk of hypertension. J Hum Hypertens, 2001. 15(5): p. 307-12.
40. Pi-Sunyer, X., A clinical view of the obesity problem. Science, 2003. 299(5608): p. 859-60.
41. Zhu, G., et al., NPY Leu7Pro and alcohol dependence in Finnish and Swedish populations. Alcohol Clin Exp Res, 2003. 27(1): p. 19-24.
42. Karvonen, M.K., et al., Association of a leucine(7)-to-proline(7) polymorphism in the signal peptide of neuropeptide Y with high serum cholesterol and LDL cholesterol levels. Nat Med, 1998. 4(12): p. 1434-7.
43. Niskanen, L., et al., Leucine 7 to proline 7 polymorphism in the neuropeptide Y gene is associated with enhanced carotid atherosclerosis in elderly patients with type 2 diabetes and control subjects. J Clin Endocrinol Metab, 2000. 85(6): p. 2266-9.
44. Dallman, M.F., et al., Chronic stress and obesity: a new view of "comfort food". Proc Natl Acad Sci U S A, 2003. 100(20): p. 11696-701.
45. Schreyer, S.A., D.L. Wilson; and R.C. LeBoeuf, C57BL/6 mice fed high fat diets as models for diabetes-accelerated atherosclerosis. Atherosclerosis, 1998. 136(1): p. 17-24.
46. Valet, P., et al., Neuropeptide Y and peptide YY inhibit lipolysis in human and dog fat cells through a pertussis toxin-sensitive G protein. J Clin Invest, 1990. 85(1): p. 291-5.
47. Kessler, J.A., G. Conn, and V.B. Hatcher, Isolated plasma membranes regulate neurotransmitter expression and facilitate effects of a soluble brain cholinergic factor. Proc Natl Acad Sci U S A, 1986. 83(10): p. 3528-32.
48. Turtzo, L.C., R. Marx, and M.D. Lane, Cross-talk between sympathetic neurons and adipocytes in coculture. Proc Natl Acad Sci U S A, 2001. 98(22): p. 12385- 90.
49. Turtzo, L.C. and M.D. Lane, Completing the loop: neuron-adipocyte interactions and the control of energy homeostasis. Horm Metab Res, 2002. 34(11-12): p. 607-15.
50. Kerekes, N., et al., Effect of NGF, BDNF, bFGF, aFGF and cell density on NPY expression in cultured rat dorsal root ganglion neurones. J Auton Nerv Syst, 2000. 81(1-3): p. 128-38.
51. Peeraully, M.R., J.R. Jenkins, and P. Trayhurn, Nerve growth factor gene expression and secretion in white adipose tissue: Regulation in 3T3-L1 adipocytes by hormones and inflammatory cytokines. Am J Physiol Endocrinol Metab, 2004.
52. Uddman, R., et al., Neuropeptide Y Y1 and neuropeptide Y Y2 receptors in human cardiovascular tissues. Peptides, 2002. 23(5): p. 927-34.
53. Marsh, D.J., et al., Role of the Y5 neuropeptide Y receptor in feeding and obesity. Nat Med, 1998. 4(6): p. 718-21.
54. Rubin, C.S., et al., Development of hormone receptors and hormonal responsiveness in vitro. Insulin receptors and insulin sensitivity in the preadipocyte and adipocyte forms of 3T3-L1 cells. J Biol Chem, 1978. 253(20): p. 7570-8.
55. Park, H.Y., et al., Potential role of leptin in angiogenesis: leptin induces endothelial cell proliferation and expression of matrix metalloproteinases in vivo and in vitro. Exp Mol Med, 2001. 33(2): p. 95-102.
56. Cantarella, G., et al., Nerve growth factor-endothelial cell interaction leads to angiogenesis in vitro and in vivo. Faseb J, 2002. 16(10): p. 1307-9.
57. Kraemer, R. and B.L. Hempstead, Neurotrophins: novel mediators of angiogenesis. Front Biosci, 2003. 8: p. s1181-6.
58. Nakahashi, T., et al., Vascular endothelial cells synthesize and secrete brainderived neurotrophic factor. FEBS:Left, 2000. 470(2): p. 113-7.
59. Calza, L., et al., Nerve growth factor control of neuronal expression of angiogenetic and vasoactive factors. Proc Natl Acad Sci U S A, 2001. 98(7): p. 4160-5.
60. Beck-Sickinger, A.G., et al., Cyclopeptide analogs for characterization of the neuropeptide Y Y2-receptor. J Recept Res, 1993. 13(1-4): p. 215-28.
61. Demuth, H.U., Hoffmann, T., Glund, K., McIntosh, C.H.S., Pederson, R.A., Fuecker, K., Fischer, S., Hanefeld, M., Single Dose Treatment of Diabetic Patients by the DPIV Inhibitor P32/98. Diabetes, 2000(49): p. Suppl. 1, A102.
62. Erickson, J.C., G. Hollopeter, and R.D. Palmiter, Attenuation of the obesity syndrome of ob/ob mice by the loss of neuropeptide Y. Science, 1996. 274(5293): p. 1704-7.
63. Mandrup, S., et al., Obese gene expression at in vivo levels by fat pads derived from s.c. implanted 3T3-F442A preadipocytes. Proc Natl Acad Sci U S A, 1997. 94(9): p. 4300-5.
64. Segal-Lieberman, G., et al., NPY ablution in C57BL/6 mice leads to mild obesity and to an impaired refeeding response to fasting. Am J Physiol Endocrinol Metab, 2003. 284(6): p. E1131-9.
65. Hutley, L.J., et al., Human adipose tissue endothelial cells promote preadipocyte proliferation. Am J Physiol Endocrinol Metab, 2001. 281(5): p. E1037-44.
66. Halvorsen, Y.C., W.O. Wilkison, and J.M. Gimble, Adipose-derived stromal cells--their utility and potential in bone formation. Int J Obes Relat Metab Disord, 2000. 24 Suppl 4: p. S41-4.
67. Marek, K.L. and R.E. Mains, Biosenthesis, development, and regulation of neuropeptide Y in superior cervical ganglion culture. J Neurochem, 1989. 52(6): p. 1807-16.
68. Paquet, L., B. Massie, and R.E. Mains, Proneuropeptide Y processing in large dense-core vesicles: manipulation of prohormone convertase expression in sympathetic neurons using adenoviruses. J Neurosci, 1996. 16(3): p. 964-73.
69. Wilson, M.J., et al., Dipeptidylpeptidase IV activities are elevated in prostate cancers and adjacent benign hyperplastic glands. J Androl, 2000. 21(2): p. 220-6.
70. Tang, Q.Q. and M.D. Lane, Activation and centromeric localization of CCAAT/enhancer-binding proteins during the mitotic clonal expansion of adipocyte differentiation. Genes Dev, 1999. 13(17): p. 2231-41.
71. Kaestner, K.H., et al., Transcriptional repression of the mouse insulin-responsive glucose transporter (GLUT4) gene by cAMP. Proc Natl Acad Sci U S A, 1991. 88(5): p. 1933-7.
72. Zhang, C., et al., Inflammation is involved in the organ damage induced by sinoaortic denervation in rats. J Hypertens, 2003. 21(11): p. 2141-8.
73. Demas, G.E. and T.J. Bartness, Novel method for localized, functional sympathetic nervous system denervation of peripheral tissue using guanethidine. J Neurosci Methods, 2001. 112(1): p. 21-8.
74. Shklyaev, S., et al., Sustained peripheral expression of transgene adiponectin offsets the development of diet-induced obesity in rats. Proc Natl Acad Sci U S A, 2003. 100(24): p. 1421:7-22.

### Reference EXAMPLE 4 Assessment of Effects of NPY on Endothelial Cell and Preadipocyte Proliferation and Adipocyte Differentiation

Stress has been linked to the pathogenesis of many diseases, yet its mechanisms of action and role as a risk factor remain problematic. The lack of a precise definition and ubiquitous presence of stress make it, like aging, an unavoidable, yet not necessarily pathogenic, fact of life. Its effects depend on its type, intensity and duration, prior exposure and the genetic makeup of the individual. The rise in stress has paralleled the incidence of obesity; however their relationship remains unclear: some people lose weight when stressed, while others gain. This has been viewed as "a matter of the mind" - due to differences in perception of stress or differential coping patterns, i.e. alterations in food intake, sympathetic nerve activity and/or hypothalamic-pituitary-adrenocortical (HPA) function. The question remains: is the stress-body relationship only in the "mind"?

Human obesity is often associated with increased sympathetic activity¹. (References cited in this example are those which follow the example.) However, sympathetic release of norepinephrine (NE) and activation of its β-adrenergic receptor promotes weight *loss* by stimulating lipolysis in white (WAT) and thermogenesis in brown adipose tissue (BAT)² and inhibiting adipocyte proliferation³. Hence, in obesity, β-adrenergic activity may be offset by more powerful mechanisms which promote weight gain. One possible factor is neuropeptide Y (NPY), a brain- and sympathetic nerve-derived peptide with potent orexigenic activity⁴. Increased brain activity of NPY and its multiple receptors (Rs), Y1, Y2 and Y5, was found in many forms of experimental obesity⁴. In ob/ob mice which lack leptin, a fat-derived hormone that inhibits appetite and hypothalamic NPY release⁵, knocking out the Y2R decreases their hyperphagia and body weight, and improves metabolism⁶. While this supports central effects of NPY-Y2 on energy homeostasis, peripheral mechanisms have not been examined. Thus, Applicants specifically focused on the role of Y2Rs in adipose tissue itself.

WAT is a well-vascularized, innervated, and hormonally active tissue, which undergoes rapid remodeling during starvation or caloric overload. Local production of fat depot-specific hormones such as visfatin⁷ or glucocorticoids⁸, and inflammation⁹ have already been linked to visceral obesity and metabolic syndrome. The essential role of WAT vascularization was, first demonstrated by Rupnick et al.¹⁰ who used anti-angiogenic therapy to reduce fat and weight. Others¹¹ confirmed that adipogenesis follows angiogenesis and proposed new strategies to induce WAT atrophy. Physiological WAT remodeling mechanisms and the role of nerves in the angiogenesis-adipogenesis cycle, however, have not been determined.

NPY acts peripherally as a sympathetic vasoconstrictor co-transmitter, released by various stressors¹². NPY is also expressed non-neuronally in endothelium¹³ and macrophages¹⁴ The peptide modulates immune responses¹⁵ and growth of various cells in a receptor-specific manner¹⁶. Through its endothelial and monocyte Y2Rs, it stimulates angiogenesis and plays a major role in neovascularization of ischemic tissues¹³, retinopathy¹⁵, wound healing¹³ and tumors¹⁶. Therefore, Applicants hypothesized that NPY exhibits similar growth-promoting and immuno-modulatory activities in WAT, and its sympathetic release and activation of Y2Rs are an endogenous mechanism regulating angiogenesis, adipogenesis, and stress-induced obesity.

Work described in this example demonstrates that NPY stimulates endothelial cell and preadipocyte proliferation, as well as adipocyte differentiation. Turtzo et al¹ have previously determined that NPY is anti-lipolytic and opposes the action of catecholamines in human adipocytes. When they are co-cultured with sympathetic neurons, neuronal NPY expression increases and neurotransmission shifts towards lipid storage and away from β-adrenergic lipolysis¹. To determine if neuronally-derived NPY promotes growth and differentiation of preadipocytes, Applicants cocultured sympathetic neuron-derived tumor cells (neuroblastomas), which express and release NPY, with 3T3-L1 preadipocytes and endothelial cells, which express Y2Rs. Both NPY and neuroblastoma-conditioned media stimulated preadipocyte and endothelial cell proliferation, which was blocked by Y2R antagonist (BIIE0246 1 µM), while neuroblastoma co-cultured with preadipocytes or endothelial cells markedly up-regulated their Y2Rs. NPY expression in neuroblastoma cells was unaffected by co-culture but was markedly up-regulated by dexamethasone (10µM, 24hrs), while tyrosine hydroxylase (TH) mRNA decreased. Similar glucocorticoid-mediated induction of NPY expression in neuronal cells was reported by others¹⁷ and suggests that during stress, elevated glucocorticoids may switch sympathetic transmission to favor NPY production over TH-dependent norepinephrine/epinephrine production.

In addition to being proliferative for preadipocytes and endothelial cells via Y2Rs, NPY mimicked insulin effects by stimulating adipogenesis in preadipocytes primed for differentiation. NPY increased leptin and resistin secretion, and lipid filling, and these effects were blocked by an Y2R antagonis. Thus, preadipocyte and endothelial Y2Rs (with other NPY-Rs potentially playing an auxiliary role¹³) are a major pathway by which NPY stimulates angiogenesis and adipogenesis *in vitro.*

### Work presented herein also shows that NPY increases subcutaneous abdominal fat, while Y2R antagonist decreases subcutaneous abdominal fat

To determine if IVPY-Y2R increases WAT mass *in vivo*, Applicant used genetically obese ob/ob mice, known for their centrally-mediated hyperphagia, impaired metabolism and reduced sympatho-adrenergic activity⁴. Unexpectedly, these mice had 3-fold higher plasma NPY levels and markedly up-regulated NPY and Y2R expression in subcutaneous abdominal fat, compared to controls--supporting a fat-derived origin of circulating peptide. NPY delivery (1 µg/14 day-pellet, previously found to produce localized angiogenesis¹³) into subcutaneous abdominal fat, significantly increased adipose tissue weight(g) and volume (measured by magnetic resonance imaging, MRI) in both obese and lean mice. Conversely, Y2R antagonist (BIIE0246 1 µM/day), injected s.c. into the abdominal pads, decreased their weight and volume by 50% within 2-weeks. These results suggest that NPY potently stimulates *in situ* adipogenesis via Y2Rs.

Mice deficient in endothelial nitric oxide synthase (eNOS^{-/-}), which are resistant to NPY-mediated angiogenesis¹³, were found to be resistant to NPY's adipogenic activity, suggesting that adipogenesis is dependent on angiogenesis. This was confirmed by the effects of Y2R antagonist, whose intra-fat injections decreased the density of von Willebrand-positive microvessels in parallel to increasing TUNEL-positive staining of endothelial cells and adipocytes, indicating anti-angiogenic and apoptotic effects of the antagonist for these cells, which, in turn, induced fat "melting". Thus, endothelial and preadipocyte expression of Y2Rs mediate fat remodeling in lean and ob/ob mice, and up-regulation of the NPY pathway leads to fat growth via an angiogenesis-dependent mechanism.

A similar system appears to exist in human adipose tissue, which expresses NPY and Y2Rs in adipocytes and endothelial cells (Fig.S2). An NPY pellet (1 µg/14 days) administered locally alongside a human liposuction-derived fat xenograft in athymic mice, increased its vascularization (density of CD3 1 + vessels) and 3-month survival, while the placebo-treated grafts underwent resorption (Fig. 2G, H).

### Stress and a high-fat/sugar diet create a mouse model of metabolic syndrome

Leptin deficiency rarely happen in humans. Therefore, results obtained with the ob/ob model were also confirmed using a more common paradigm leading to obesity: a high fat/high sugar diet (HF). Dallman's group has shown that under chronic stress, rats prefer a HF 'comfort food' diet which appears to inhibit brain levels of corticotropin-releasing factor, a potent anxiogenic and "master" stress hormone activating both the HPA-axis and sympathetic nerves¹⁸. Using a modified version of Dallman's 'comfort food' regimen combined with chronic intermittent cold exposure, Applicants created a "human-like" obesity model where NPY's role was determined.

Cold stress, like food intake, stimulates sympathetic nerve activity, thermogenesis¹⁹ and is a powerful stimulus for NPY release¹². Mice were stressed (ST) for 2-weeks to 3-months (1 hr/day standing in 0.5 cm of ice-cold water), and fed either a standard or HF diet. After 14 days, HF alone increased interscapular BAT, sternal and abdominal (subcutaneous and visceral) WAT depots, and increased food intake). Stress alone had no effect, but combined with HF, markedly increased the abdominal fat depot and core temperature without changing body weight, food intake, urine and fecal output, compared to HF-fed or ST mice. Expression of uncoupling proteins (UCP), markers of thermogenesis¹⁴: UCP-1 in BAT, UCP-2 in WAT and UCP-3 in skeletal muscle were increased. Stress in HF-fed but not standard diet fed mice also induced a BAT-specific UCP-1 and BAT-like morphology in WAT - indicating white-to-brown fat transformation and enhanced thermogenesis. Such transformation into 'fast-oxidizing' WAT was previously observed with cold-induced β-adrenergic activation or ectopic expression of UCP-1²⁰.

In stressed HF-fed mice, subcutaneous intra-fat Y2R antagonist administration (slow-release pellet 10 µg/14days or daily injections 1µM), reduced the abdominal (subcutaneous and visceral) depot by 40% in 2-weeks - without significantly changing other depots. The essential role of Y2Rs in stress-induced obesity was further documented when the Y2R knockout abolished the stress effect and normalized abdominal fat volumes to levels seen in HF-fed mice.

Stress in HF-fed mice also markedly up-regulated NPY, Y2 and DPPIV/CD26 expression in the subcutaneous abdominal pad, suggesting that all the components of the peptide's angiogenic system are activated. In contrast, HF alone mainly increased DPPIV/CD26. DPPIV/CD26 is an endothelial enzyme which converts NPY to NPY3-36, a selective Y2/Y5 agonist that is critical for NPY-mediated angiogenesis ¹⁵. It also inactivates insulin-sensitizing glucagon-like peptide-1 (GLP- 1)²¹. Therefore, DPPIV inhibitors are being developed as anti-diabetic drugs. Thus, HF-induced up-regulation of DPPIV expression may contribute to obesity and metabolic derangements by inactivating GLP-1, while activating angiogenic and adipogenic NPY pathway. Supporting this notion is the resistance of cd26^{-/-} mice to diet-induced obesity²².

### Stress and a high-fat/sugar diet induces morphological changes in fat

Stress in HF-fed mice also changed the morphology of WAT by increasing its cellularity due to the proliferation of small adipocytes. This effect was mimicked by NPY in WT mice, and reversed by Y2R antagonist, suggesting that stress via the NPY-Y2 system stimulates the proliferation of new adipocytes, as seen *in vitro* in 3T3-L1 preadipocytes. Although the origin of new adipocytes remains undetermined, they may be derived from nonadipocyte pluripotential stromal cells, which can differentiate into adipocytes, endothelial cells, and macrophages²⁰. These three cell types were increased by stress in the abdominal fat, and strongly expressed NPY - indicating neuronal as well as extraneuronal NPY origin in WAT. Applicants previously found auxiliary peptide expression in states of active remodeling during ischemia¹³, retinopathy¹⁵, atherosclerosis¹⁴ or immune cell activation²³. Induction of NPY in extraneuronal sources may explain massive increases in WAT mass following sympathectomy, previously attributed mostly to the loss of fat-wasting sympathetic adrenergic effects³.

Stress and a HF-diet also up-regulated endothelial Y2R expression, localized to the areas of increased adipose tissue vascularization (CD31+ vessels), inflammation (CD68+ macrophages), and adipocyte proliferation. These changes suggest that stress, via NPY-Y2R, activates adipose tissue monocyte/macrophage infiltration, which have been previously shown' to be associated with both adipose tissue growth and angiogenesis²². Local Y2R antagonist treatment inhibited adipocyte proliferation (Ki67+ staining) and increased their apoptosis (TUNEL) - indicating the critical role of this receptor in inflammatory and proliferative responses of WAT during remodeling.

### Chronic stress induces metabolic dysfunction in HF-fed mice and Y2R antagonist inhibits the symptoms

Preferential abdominal fat accumulation and NPY/Y2/DPPIV activation in chronically stressed mice suggests that stress, via this pathway, promotes the development of metabolic syndrome. Alone, stress elevated plasma NPY and resistin levels; HF decreased NPY, but increased leptin. An inhibitory leptin-NPY relationship in the periphery mirrors their inhibitory interaction in hypothalamic appetite control. Stress potentiated the effects of a HF-diet and further elevated resistin, an adipokine known to promote insulin resistance²⁴. Two weeks of stress and a HF-diet decreased insulin levels and glucose tolerance was impaired. This could be due to increased resistin, which has been implicated in glucose intolerance²⁵, as well as stress- and diet-induced changes in glucocorticoids. By 3-months, insulin, leptin and resistin levels were all significantly elevated in HF+ST mice, indicating insulin resistance. Three months of stress and HF induced gross obesity, with preferential abdominal and visceral fat accumulation and increased variability and mean level of arterial blood pressure (telemetry) suggesting development of hypertension and metabolic syndrome.

In humans, abdominal obesity is associated with hypercortisolemia only in Cushing's syndrome, but not in diet-induced obesity²⁶. Similarly, in stress- and HF-diet-induced obesity, plasma corticosterone was not elevated. However, both HF and stress (but not stress alone) increased corticosterone levels in subcutaneous abdominal fat. Abdominal visceral fat (but not other depots) is known to produce glucocorticoids *in situ* from inactive 11-keto-forms, due to the site-specific expression of 11 β-hydroxysteroid dehydrogenase type-1 (11βHSD-1)⁸. This enzyme was up-regulated by stress alone. However, when stress was combined with HF-diet, 11βHSD-1 expression fell below control levels. This may indicate that elevated adipose tissue-derived glucocorticoids feedback inhibit enzyme synthesis as a part of an adaptive response counteracting metabolic abnormalities induced by a HF-diet²⁷.

Given that glucocorticoids up-regulate NPY expression in many tissues^{28,29}, their effects in WAT may favor a high NPY state and contribute to metabolic derangements. The opposite occurs with Y2R deficiency in ob/ob mice which attenuates HPA activity and type II diabetes⁶. As shown *in vitro*, sympathetic neuron-derived neuroblastoma cells favors NPY expression following glucocorticoid (dexamethasone, 10µM/24hr) treatment. It appears that HF and stress act similarly and by increasing corticosterone production in the abdominal fat, up-regulate NPY expression in the sympathetic nerves (and other non-neuronal sources) of this depot. While NPY release is being favored, HF and stress significantly reduced plasma epinephrine levels, without altering plasma norepinephrine. With the increased ratio of NPY-to-epinephrine plasma levels, stress appears to shift adipose tissue metabolism towards NPY-mediated adipogenesis, and away from β-adrenergic lipolysis, and hence, promote weight gain. Intra-fat injection of Y2 antagonist reversed this imbalance and, in spite of still elevated corticosterone levels in the abdominal fat, reduced fat mass and normalized glucose intolerance. Thus, glucocoticoids are up-stream regulators of NPY expression, and Y2 adipogenic/angiogenic system mediates, at least in part, pro-adipogenic effects of glucocorticoids.

A HF-diet also led to steatosis in the liver and skeletal muscle, hyperlipidemia, and abdominal fat accumulation, and these effects were dramatically increased by stress. Surprisingly, local intra-fat injections of Y2R antagonist within 2-weeks normalized plasma adipokine and NPY levels, increased insulin levels, improved metabolic profile and inhibited tissue steatosis. How intra-fat injections of Y2R antagonist could have such systemic effects is puzzling. The antagonist could act either directly on β-cells, via circulation, or indirectly, by reducing fat and secretion of some yet unidentified anti-insulin factors (possibly, resistin). The former is likely as pancreatic Y2Rs were shown to inhibit insulintelease^{6,28}. Alternatively, reduction of fat depots may act indirectly, similarly to beneficial (although short-lasting) effects of large liposuction³⁰.

These data, together with the insulin-like effects of NPY on 3T3-L1 adipogenesis suggest that NPY acts as an insulin-mimetic on actions such as adipocyte differentiation, and resistin and leptin secretion. However, NPY also opposes insulin since it promotes glucose intolerance¹⁵, hyperlipidemia^{14,15}, hypertension¹², and is pro-atherogenic and pro-inflammatory¹⁴. Glucocorticoids and sympathetic nerves were previously shown to switch β-cell secretion from insulin to NPY²⁸, and now Applicants have shown the same mechanism in sympathetic neuron-derived neuroblastoma cells switching from TH to NPY (Fig. 1B), suggesting that a similar process may occur during HF-diet feeding and chronic stress. Remarkably, this stress-induced insulin-to-NPY switch and metabolic abnormalities were fully prevented by intra-fat administration of Y2R antagonist.

### DISCUSSION

The crucial question arises, "where does the melted fat go?" Intra-fat injections of Y2R antagonist did not alter stress+HF-induced hyperlipidemia or UCP-1/2 expression in WAT and it also reduced liver and skeletal muscle steatosis. Bones may be another alternative long-term energy storage site. Like fat, bones are continually remodeled and, interestingly, knockout of Y2Rs was found to increase, whereas sympathetic β-adrenergic hyperactivity decreases, bone density, while both reduce fat mass³¹. Stress alone reduced bone density (preliminary unpublished data) and when combined with HF, lowered plasma osteocalcin levels, suggesting bone loss. Remarkably this response was completely normalized by intra-fat Y2R antagonist delivery. These data indicate that the Y2R antagonist shifts the energy storage from WAT to the bones. Such an action would greatly benefit patients by preventing not only weight gain - but also age-associated bone loss.

Three months of stress and a HF-diet resulted in abdominal/overall obesity, glucose intolerance, hyperinsulinemia, and hypertension - hallmarks of the metabolic syndrome⁹. Marked activation of the NPY-Y2Rs in WAT suggests that this pathway is a biomarker for this condition. Of particular risk for such stress-dependent obesity may be people with a common NPY-signal peptide gene polymorphism, who reportedly have greater stress-induced peptide release, as well as atherosclerosis and diabetic retinopathy¹⁵. It is reasonable to conclude that high NPY levels are a risk factor for stress-induced obesity and metabolic syndrome by activating the NPY-Y2 angiogenic/adipogenic and proinflammatory pathway, and mice stressed and HF-fed represent a 'human-like' model of this disease.

Applicants conclude that NPY and its Y2Rs are responsible for, while Y2R deficiency protects against, stress-induced abdominal obesity. The NPY-Y2 system acts indirectly via angiogenesis and directly, by modulating preadipocytes/adipocyte proliferation, apoptosis and differentiation. The HF-diet increases corticosterone levels in plasma and abdominal fat and this primes sympathetic nerves and NPY-ergic cells in WAT to favor NPY production. Cold-stress further promotes an overall high-NPY state by inducing peptide expression in neural, endothelial and immune cells, which further stimulates adipogenesis, glucose intolerance, and abdominal fat accumulation. Although visceral fat is primarily linked to cardiovascular pathology, abdominal subcutaneous fat may be equally dangerous, as up to 80% of the fatty acids entering the liver are derived from that area²⁴.

Since stress-induced amplification of adiposity and obesity was absent in Y2 antagonist-treated or Y2R-deficient mice, this opens new avenues for the treatment of obesity and metabolic syndrome with Y2R selective antagonists, which in addition to reducing abdominal fat, improves metabolism and builds better bones. A common silent Y2R gene variant which was recently found in Swedes³² protecting them against diet-induced obesity provides a proof-of-concept in humans of the anti-adipogenic actions of Y2R inhibition. Fat-targeted blockade of the adipose tissue-derived NPY-Y2 pathway may also complement the anti-obesity actions of centrally acting appetite-inhibitory drugs. While the popularity of intra-fat administration of compounds for fat melting or augmentation, known as mesotherapy³³, has been increasing, currently, there are no scientifically proven compounds approved for use in humans for remodeling fat tissue. This study is the first to demonstrate that NPY-Y2R-based drugs may be useful for mesotherapy for health and cosmetic applications. It also provides a new mouse model of metabolic syndrome which resembles the present human condition of daily stress and hypercaloric diets.

The following methods and materials were used in Example 4.

### Methods

**1. Cell culture:** 3T3-L1 preadipocyte cells (gift from Dr. Lane, ³⁴) were cultured in DMEM high glucose media with glutamine, 10% calf sera (Gemini Bioproducts), d-Biotin (8µg/ml) and Pantothenate (8µg/ml) (Sigma). SK-N-BE(2) neuroblastoma cells (ATCC) were cultured in EMEM/F12K with 10% fetal bovine sera (FBS). HMVEC endothelial cells (Cambrex) were cultured in endothelial cell growth media (Cambrex). All media was supplemented with 100 units/ml penicillin, 100 µg/ml streptomycin and 2.5 mg/ml amphotericin.
**2. Proliferation assay**: Cells were plated onto 96-well dishes, growth-arrested in serum-free media for 24 h, and then were treated with the desired factors for 24 h in the culture media supplemented with 0.25% FBS; 4 hr after treatment 0.5µCi [³H] thymidine was added to each well. After 24 h, cells were harvested in a 96-well harvester (Tomtec) and counted in a Betaplate Liquid Scintillation Counter (Wallac).
**3. Co-culture**: For mitogenic assay and expression studies, co-culture was performed in a Costar Transwell system. Cells were plated on 6-well plates or inserts with 0.4µm Polyester membranes. At 30% confluence, the inserts were combined with wells containing other cells. Co-culture of neuroblastomas with endothelial cells (EC) were carried out in EC media and preadipocytes in preadipocyte growth media. After the respective treatment, the cells growing on the plates were harvested for RNA isolation. For the mitogenic assay, cells were first growth-arrested in serum-free media for 24 hr, then the inserts placed into the plates and cells treated with desired factors in 0.25% FBS. After 48 hr, the cells were counted with a Beckman Coulter Counter. In both co-culture models, media was collected for Leptin (R&D Systems), Resistin (R&D Systems), and NPY (Bachem) ELISA.
**4. Real-time RT-PCR**: RNA was isolated using Tri Reagent (Sigma) and cDNA synthesized by iScript cDNA synthesis kit (BioRad). ICycler iQ Detection System (BioRad) was used to perform Real Time PCR. cDNA was amplified for 40 cycles using TaqMan PCR Reagent Kit and pre-designed primers and fluorescein-labeled probes from Applied Biosystems, as previously described ¹⁶, according to the manufacturer's procedure. The results were analyzed using software provided by BioRad and expression levels calculated by the comparative C_{T} method using β-actin as an endogenous reference gene, according to the Applied Biosystems' ABI PRISM 7700 User Bulletin #2.
**5. TUNEL:** TUNEL reaction was performed using In Situ Cell Detection Kit (Roche Diagnostic) and signal converted to visible light using AP-converter. The density of TUNEL positive cells will be measured using NIH ImageJ software (using a plugin written by Wayne Rasband).
**6. ELISA**: ELISA kits for: NPY (Bachem Laboratories), mouse Leptin, Corticosterone, and Resistin (R&D systems), rat/mouse Insulin and mouse Adiponectin (Linco Research), and mouse Osteocalcin (Biomedical Technologies, Inc.) were used. The assays were performed according to the manufacturers' procedures.
**7. Immunohistochemistry:** Immunostaining was performed using the following primary antibodies: rabbit polyclonal anti-NPY (Bachem Laboratories) and mouse monoclonal anti-TH (Immunostar), rabbit anti-Y2R (AstraZeneca), goat anti-DPPIV/CD26 (R&D systems), rat anti-mouse CD31 (BD Pharmigen), mouse monoclonal anti-CD31 (Abcam), rabbit anti-human vWF (DAKO), mouse anti-human CD68 (DAKO) and rabbit polyclonal anti-mouse Ki67 (DAKO).
**8. Use of animals and human samples**. The use of animals and human tissue samples in this study was approved by the Institutional Animal Care and Use Committee and the Institutional Review Board, respectively, at the Georgetown University Medical Center and Hospital.
**9. Human fat implantation**: Human fat from liposuction was received from Dr. Stephen Baker's surgical team (Georgetown University Medical Center/MedStar Health) and loaded into 1 ml syringes. Athymic nude mice (Taconic) were anesthetized and slow-release pellets inserted subcutaneously. 100cc of fat was implanted in close proximity to the pellets using an 18 gauge Coleman infiltration cannula (Byron Medical) attached to the syringe. The skin wound was closed with medical cyanoacrylate.
**10. Ultrasonography**: Mice were anesthetized by isoflurane 1-3% in oxygen and a sterile water based ultrasonic gel was applied to the area (Aquasonic gel - Visual Sonics). The mice were placed in the Visual Sonics mouse holder containing a thermostatically controlled heating pad to maintain mouse body temperature. The imaging was performed with a small animal ultrasound system, Visual Sonics Vivo 660 (55 MHz).
**11. MRI:** A Brukker 7-Tesla small-animal magnetic resonance imager coil was used to visualize and noninvasively quantitate various fat depots. A 3-dimensional (3D) T2-weighted (T2W) imaging protocol optimized for high contrast fat-imaging was implemented. This 3-D T2W RARE (Rapid Acquisition with Relaxation Enhancement) imaging sequence TE 5.9, TR 200, Rare Factor 8, Matrix 256X128X128, 7X3X3cm - 9X3X5cm (Cranial-Caudal x AP x LR) produced a reconstructed image that shows fat as the brightest signal while signals from other tissues were relatively suppressed. Quantification of the total body fat and separate specific fat depots were calculated using thresholding and voxel count plugins (by Wayne Rasband) from NIH imageJ software. VolumeJ plugin (by Michael Abramoff) was used to create 3D fat-images. The animal management system that was used in conjunction with the MRI was used to record core, skin, ambient and water blanket temperature measurements that are monitored during the imaging. The water blanket was used to regulate core temperature of the animal during imaging and anesthesia.
**12. Oil Red-O staining:** Oil Red O solution (0.36% Oil Red O solution in 60% isopropanol) was used to stain lipid droplets in mature adipocytes and paraffin embedded tissues (Chemicon Adipogenesis Assay kit). Oil Red-O in propylene glycol (Newcomer Supply) was used to stain lipids in liver and muscle frozen sections.
**13. High fat diet:** Male C57BL/6, SV129J (both from JAX laboratories), Y2R^{-/-} mice (gift from Dr. Herzog and AstraZeneca) 6-8 weeks old were given either a standard chow (SC) diet: 28% protein, 60% carbohydrate, 12% fat (4 kcal/gm) or a high fat /high sugar (HF) diet: 20% protein, 35% carbohydrate, 45% fat (4.7 kcal/gm) from Research Diets for 14 days - 3 months.
**14. Stress**: Cold-stress was carried out as previously described ¹². Briefly, mice were placed in 0.5 cm ice water for 1 hr for 14 days - 3 months.
**15. Glucose tolerance test:** After an overnight 17 hr fast, unanesthetized mice (JAX laboratories) were injected i.p. with a dose of 1.5 g of 50% glucose solution per kg of body weight. Blood samples were obtained from the tail vein 30 min prior to the glucose challenge and then again at 0, 30, 60, 90 min after the glucose challenge. Blood glucose concentrations were measured with a FreeStyle portable glucose meter (TheraSense).
**16. Blood pressure:** Mice were implanted with transmitters from Data Sciences International (DSI) Physiotel® telemetry system. Mice were allowed to recover for 5 days and then 24 hr baseline recordings were taken followed by 3 days of recording. Acquisition software was used to calculate diastolic, systolic, and mean arterial pressure.
**17. Statistical analysis:** All comparisons were subject to a two-tailed Student t-test with *P* ≤0.05 considered statistically significant.

**Materials:** NPY and its derivatives were purchased from Bachem Laboratories. NPY R antagonists: Y1 - H409/22 acetate (gift from Astra Zeneca), Y2 - BIIE0246 (Tocris), Y5 - L-152,804 (Tocris). Slow release pellets containing desired factors were purchased from Innovative Research of America.

**References** The following references are those cited in Example 4.
1. Turtzo, L.C. & Lane, M.D. Completing the loop: neuron-adipocyte interactions and the control: of energy homeostasis. Horm Metab Res 34, 607-15 (2002).
2. Bachman, E.S. et al. betaAR signaling required for diet-induced thermogenesis and obesity resistance. Science 297, 843-5 (2002).
3. Bowers, R.R. et al. Sympathetic innervation of white adipose tissue and its regulation of fat cell number. Am J Physiol Regul Integr Comp Physiol 286, R1167-75 (2004).
4. Kalra, S.P. & Kalra, P.S. NPY and cohorts in regulating appetite, obesity and metabolic syndrome: beneficial effects of gene therapy. Neuropeptides 38, 201-11 (2004).
5. Mark, A.L., Correia, M.L., Rahmouni, K. & Haynes, W.G. Selective leptin resistance: a new concept in leptin physiology with cardiovascular implications. J Hypertens 20, 1245-50 (2002).
6. Sainsbury, A., Schwarzer, C., Gouzens, M. & Herzog, H. Y2 receptor deletion attenuates the type 2 diabetic syndrome of ob/ob mice. Diabetes 51, 3420-7 (2002).
7. Fukuhara, A. et al. Visfatin: a protein secreted by visceral fat that mimics the effects of insulin. Science 307, 426-30 (2005).
8. Masuzaki, H. et al. A transgenic model of visceral obesity and the metabolic syndrome. Science 294, 2166-70 (2001).
9. Dandona, P., Aljada, A., Chaudhuri, A., Mohanty, P. & Garg, R. Metabolic syndrome: a comprehensive perspective based on interactions between obesity, diabetes, and inflammation. Circulation 111, 1448-54 (2005).
10. Rupnick, M.A. et al. Adipose tissue mass can be regulated through the vasculature. Proc Natl Acad Sci USA 99, 10730-5 (2002).
11. Brakenhielm, E. et al. Angiogenesis inhibitor, TNP-470, prevents diet-induced and genetic obesity in mice. Circ Res 94, 1579-88 (2004).
12. Zukowska-Grojec, Z. Neuropeptide Y. A novel sympathetic stress hormone and more. Ann N Y Acad Sci 771, 219-33 (1995).
13. Zukowska, Z., Grant, D.S. & Lee, E.W. Neuropeptide Y: a novel mechanism for ischemic angiogenesis. Trends Cardiovasc Med 13, 86-92 (2003).
14. Li, L., Jonsson-Rylander, A.C., Abe, K. & Zukowska, Z. Chronic stress induces rapid occlusion of angioplasty-injured rat carotid artery by activating neuropeptide Y and its Y1 receptors. Arterioscler Thromb Vasc Biol 25, 2075-80 (2005).
15. Koulu, M. et al. Neuropeptide Y and Y2-receptor are involved in development of diabetic retinopathy and retinal neovascularization. Ann Med 36, 232-40 (2004).
16. Kitlinska, J. et al. Differential effects of neuropeptide Y on the growth and vascularization of neural crest-derived tumors. Cancer Res 65, 1719-28 (2005).
17. DiMaggio, D.A., Farah, J.M., Jr. & Westfall, T.C. Effects of differentiation on neuropeptide-Y receptors and responses in rat pheochromocytoma cells. Endocrinology 134, 719-27 (1994).
18. Dallman, M.F. et al. Chronic stress and obesity: a new view of "comfort food". Proc Natl Acad Sci U S A 100, 11696-701 (2003).
19. Dulloo, A.G. Biomedicine. A sympathetic defense against obesity. Science 297, 780-1 (2002).
20. Rehman, J. et al. Secretion of angiogenic and antiapoptotic factors by human adipose stromal cells. Circulation 109, 1292-8 (2004).
21. Conarello, S.L. et al. Mice lacking dipeptidyl peptidase IV are protected against obesity and insulin resistance. Proc Natl Acad Sci USA 100, 6825-30 (2003).
22. Epstein, S.E. et al. Janus phenomenon: the interrelated tradeoffs inherent in therapies designed to enhance collateral formation and those designed to inhibit atherogenesis. Circulation 109, 2826-31 (2004).
23. Zukowska, Z., Pons, J., Lee, E.W. & Li, L. Neuropeptide Y: a new mediator linking sympathetic nerves, blood vessels and immune system? Can J Physiol Pharmacol 81, 89-94 (2003).
24. Klein, S. The case of visceral fat: argument for the defense. J Clin Invest 113, 1530-2 (2004).
25. Kitagawa, Y. et al. Impaired glucose tolerance is accompanied by decreased insulin sensitivity in tissues of mice implanted with cells that overexpress resistin. Diabetologia 47, 1847-53 (2004).
26. Rask, E. et al. Tissue-specific dysregulation of cortisol metabolism in human obesity. J Clin Endocrinol Metab 86, 1418-21 (2001).
27. Morton, N.M., Ramage, L. & Seckl, J.R. Down-regulation of adipose 11 beta-hydroxysteroid dehydrogenase type 1 by high-fat feeding in mice: a potential adaptive mechanism counteracting metabolic disease. Endocrinology 145, 2707-12 (2004).
28. Myrsen-Axcrona, U., Ahren, B. & Sundler, F. Modulatory role of adrenergic nerves on dexamethasone-induced islet cell NPY expression in the rat: evidence from chemical sympathectomy. Pancreas 18, 180-8 (1999).
29. Laborie, C. et al. Regulation of neuropeptide Y and its mRNA by glucocorticoids in the rat adrenal gland. Neuroendocrinology 62, 601-10 (1995).
30. Kelley, D.E. Thermodynamics, liposuction, and metabolism. N Engl J Med 350, 2542-4 (2004).
31. Baldock, P.A. et al. Hypothalamic Y2 receptors regulate bone formation. J Clin Invest 109, 915-21 (2002).
32. Lavebratt, C., Alpman, A., Persson, B., Amer, P. & Hoffstedt, J. Common neuropeptide Y2 receptor gene variant is protective against obesity among Swedish men. Int J Obes (Lond) 30, 453-9 (2006).
33. Matarasso, A. & Pfeifer, T.M. Mesotherapy for body contouring. Plast Reconstr Surg 115, 1420-4 (2005).
34. Turtzo, L.C., Marx, R. & Lane, M.D. Cross-talk between sympathetic neurons and adipocytes in coculture. Proc Natl Acad Sci USA 98, 12385-90 (2001).

### EQUIVALENTS

## Claims

1. Use of a Y receptor agonist in the preparation of a medicament for increasing the stability of a fat graft, the medicament adapted for administration to the fat graft and/or to a fat graft recipient at or proximal to the site of the graft, wherein the Y receptor agonist is one or more of the following: NPY, NPY₂₋₃₆, NPY₃₋₃₆, NPY₁₃₋₃₆, NPY18-36, or [Leu³¹, Pro³⁴]NPY, PYY₃₋₃₆,C2-NPY,[Ala³]NPY, [Ala³⁰]NPY, [Ala⁵]NPY, [Ala¹³]NPY, [Ala²⁰]NPY, [Ala²¹]NPY, [Ala²²]NPY, [Ala⁸]NPY, [Ala²⁷]NPY, [Ala²⁸]NPY, [Ala³⁶]NPY, TASP-V, N-acetyl [Leu^{28,31}]NPY²⁴⁻¹⁶, TyrIleAsnLeuIleTyrArgLeuArgTyr-NH₂, Ac[Leu^{28,31}] NPY²⁴⁻³⁶, Ac-cyclo^{28/32}[Ala²⁴,Lys²⁸,Leu³¹,Glu³²]NPY²⁴⁻³⁶,Ac-cyclo^{28/32}[Ala²⁴,Lys²⁸, Glu³²]NPY²⁴⁻³⁶, Ac-cyclo^{28/32}[Lys²⁸, Glu³²]NPY²⁵⁻³⁶,Cyclo(2/27)-des-AA⁷⁻²⁴[Glu²,Gly⁶,DDpr²⁷]NPY, Dicyclo(2/27,28/32)-des-AA⁷⁻²⁴[Glu^{2,32},DAla⁶,DDpr²⁷, Lys²⁸]NPY,[Ala(31),Aib(32)]-NPY,[Ala(31), or Pro(32)]-NPY.

2. The use of claim 1, wherein the Y receptor is a Y2 receptor or a Y5 receptor, or a Y2/Y5 receptor heterodimer.

3. The use of any preceding claim wherein the medicament comprising the Y receptor agonist is formulated in an extended release formulation or an implant.

4. The use of any preceding claim wherein the medicament comprising the Y receptor agonist is adapted for administration via injection, administered transcutaneously, delivered as an isolated drug, or in a pharmaceutical composition.

5. The use of any preceding claim wherein the Y receptor agonist is administered at least twice.

6. The use of any preceding claim wherein the fat graft is an autologous graft or a xenograft.

7. The use of any preceding claim wherein the fat graft is obtained by direct excision, liposuction or lipoaspiration.

8. The use of any preceding claim wherein the fat graft is fat tissue.

9. The use of any preceding claim further comprising administering a Y receptor agonist to the fat graft prior to implantation into the fat graft recipient.

10. The use of any preceding claim wherein the fat graft is obtained from a subcutaneous depot or a visceral depot.

11. The use of any preceding claim wherein the graft is implanted between epidermis and muscle fascia, intramuscular, supraperiosteal or subperiosteal tissue planes, or in a breast, a lip, a cheek, the face, or the forehead of the fat graft recipient.

12. The use of any preceding claim wherein the medicament is adapted for use in breast augmentation, facial reconstruction, breast reconstruction, liposuction revision, treatment of HIV protease inhibitor facial wasting; reconstructive surgery, or cosmetic surgery.

13. The use of any preceding claim wherein the Y receptor agonist is NPY or PYY.

14. Y receptor agonis for use for increasing the stability of a fat graft in an individual, wherein the Y receptor agonist is one or more of the following: NPY, NPY₂₋₃₆, NPY₃₋₃₆,NPY_{13-36,}NPY18-36, or [Leu³¹, Pro³⁴]NPY, PYY₃₋₃₆,C2-NPY,[Ala³]NPY, [Ala³⁰]NPY, [Ala⁵]NPY, [Ala¹³]NPY, [Ala²⁰]NPY, [Ala²¹]NPY, [Ala²²]NPY, [Ala⁸]NPY, [Ala²⁷]NPY, [Ala²⁸]NPY, [Ala³⁶]NPY, TASP-V, N-acetyl [Leu^{28,31}] NPY²⁴⁻³⁶, TyrIleAsnLeuIleTyrArgLeuArgTyr-NH₂, Ac[Leu^{28,31}] NPY²⁴⁻³⁶, Ac-cyclo^{28/32}[Ala²⁴,Lys²⁸,Leu³¹,Glu³²]NPY²⁴⁻³⁶,Ac-cyclo^{28/32}[Ala²⁴,Lys²⁸, Glu³²]NPY²⁴⁻³⁶, Ac-cyclo^{28/32}[Lys²⁸, Glu³²]NPY²⁵⁻³⁶,Cyclo(2/27)-des-AA⁷⁻²⁴[Glu²,Gly⁶,DDpr²⁷]NPY, Dicyclo(2/27,28/32)-des-AA⁷⁻²⁴[Glu^{2,32},DAla⁶,DDpr²⁷, Lys²⁸]NPY,[Ala(31),Aib(32)]-NPY,[Ala(31), or Pro(32)]-NPY.

15. The Y receptor for use according to claim 14, wherein Y is a Y2 receptor, a Y5 receptor or a Y2/Y5 receptor heterodimer.

## Patentansprüche

1. Verwendung eines Y-Rezeptor-Agonisten bei der Herstellung eines Medikaments zur Erhöhung der Stabilität eines Fetttransplantats, wobei das Medikament zur Verabreichung an das Fetttransplantat und/oder den Fetttransplantatempfänger am oder nahe am Ort der Transplantation hergerichtet ist, wobei der Y-Rezeptor-Agonist einer oder mehrere der folgenden ist: NPY, NPY₂₋₃₆, NPY₃₋₃₆, NPY₁₃₋₃₆, NPY18-36 oder [Leu³¹, Pro³⁴]NPY, PYY₃₋₃₆, C2-NPY,[Ala³]NPY, [Ala³⁰]NPY, [Ala⁵]NPY, [Ala¹³]NPY, [Ala²⁰]NPY, [Ala²¹]NPY, [Ala²²]NPY, [Ala⁸]NPY, [Ala²⁷,NPY, [Ala²⁸]NPY, [Ala³⁶]NPY, TASP-V, N-Acetyl [Leu^{28,31}] NPy²⁴⁻³⁶, TyrIleAsnLeuIleTyrArgLeuArgTyr-NH₂, Ac[Leu^{28,31}] NPy²⁴⁻³⁶], Ac-Cyclo^{28/32}[Ala²⁴,Lys²⁸,Leu³¹,Glu³²]NPY²⁴⁻³⁶,Ac-Cyclo^{28/32}[Ala²⁴,Lys²⁸, Glu³²]NPY²⁴⁻³⁶, Ac-Cyclo^{28/32}[Lys²⁸, Glu³²]NPy²⁵⁻³⁶, Cyclo(2/27)-des-AA⁷⁻²⁴ [Glu², Gly,DDpr²⁷]NPY, Dicyclo(2/27,28/32)-des-AA⁷⁻²⁴[Glu^{2,32},DAla⁶ ,DDpr²⁷, Lys²⁸]NPY, [Ala(31),Aib(32)]-NPY,[Ala(31) oder Pro(32)]-NPY.

2. Die Verwendung nach Anspruch 1, wobei der Y-Rezeptor ein Y2-Rezeptor oder ein Y5-Rezeptor oder ein Y2/Y5-Rezeptor-Heterodimer ist.

3. Die Verwendung nach einem vorhergehenden Anspruch, wobei das Medikament, das den Y-Rezeptor-Agonisten enthält, in einer Formulierung mit verzögerter Freisetzung oder einem Implantat formuliert ist.

4. Die Verwendung nach einem vorhergehenden Anspruch, wobei das Medikament, das den Y-Rezeptor-Agonisten enthält, hergerichtet ist zur Verabreichung durch Injektion, transkutan verabreicht wird, als ein isolierter Arzneistoff oder in einer pharmazeutischen Zusammensetzung zugeführt wird.

5. Die Verwendung nach einem vorhergehenden Anspruch, wobei der Y-Rezeptor-Agonist wenigstens zweimal verabreicht wird.

6. Die Verwendung nach einem vorhergehenden Anspruch, wobei das Fetttransplantat ein autologes Transplantat oder ein Xenotransplantat ist.

7. Die Verwendung nach einem vorhergehenden Anspruch, wobei das Fetttransplantat durch direkte Exzision, Liposuktion oder Lipoaspiration erhalten wird.

8. Die Verwendung nach einem vorhergehenden Anspruch, wobei das Fetttransplantat Fettgewebe ist.

9. Die Verwendung nach einem vorhergehenden Anspruch, ferner umfassend die Verabreichung eines Y-Rezeptor-Agonisten an das Fetttransplantat vor der Implantation in den Fetttransplantatempfänger.

10. Die Verwendung eines vorhergehenden Anspruchs, wobei das Fetttransplantat aus einem subkutanen Depot oder einem viszeralen Depot erhalten wird.

11. Die Verwendung nach einem vorhergehenden Anspruch, wobei das Transplantat zwischen Epidermis und Muskelfaszie, intramuskuläre, supraperiostale oder subperiostale Gewebeflächen oder in eine Brust, eine Lippe, eine Wange, das Gesicht oder die Stirn des Fetttransplantatempfängers implantiert wird.

12. Die Verwendung nach einem vorhergehenden Anspruch, wobei das Medikament hergerichtet ist zur Verwendung bei der Brustvergrößerung, Gesichtsrekonstruktion, Brustrekonstruktion, Liposuktionsrevision, Behandlung von fazialer HIV-Protease-Inhibitor-Lipoatrophie, rekonstruktiven Chirurgie oder kosmetischen Chirurgie.

13. Die Verwendung nach einem vorhergehenden Anspruch, wobei der Y-Rezeptor-Agonist NPY oder PYY ist.

14. Y-Rezeptor-Agonist zur Verwendung zur Erhöhung der Stabilität eines Fetttransplantats bei einem Individuum, wobei der Y-Rezeptor-Agonist einer oder mehrere der folgenden ist: NPY, NPY₂₋₃₆, NPYR₃₋₃₆, NPY₁₃₋₃₆, NPY18-36 oder [Leu³¹, Pro³⁴]NPY, PYY₃₋₃₆, C2-NPY,[Ala³]NPY, [Ala³⁰]NPY, [Ala⁵]NPY, [Ala¹³]NPY, [Ala²⁰]NPY, [Ala²¹]NPY, [Ala²²]NPY, [Ala⁸]NPY, [Ala²⁷,NPY, [Ala²⁸,NPY, [Ala³⁶]NPY, TASP-V, N-Acetyl [Leu^{28,31}] NPY²⁴⁻³⁶, TyrIleAsnLeuIleTyrArgLeuArgTyr-NH₂, Ac[Leu^{28,31}] NPY²⁴⁻³⁶], Ac-Cyclo^{28/32}[Ala²⁴,Lys²⁸,Leu³¹,Glu³²,NPY²¹⁻³⁶,Ac-Cyclo^{28/32}[Ala 2⁴,Lys²⁸, Glu³²]NPY²⁴⁻³⁶, Ac-Cyclo^{28/32}[Lys²⁸, Glu³²]NPY²⁵⁻³⁶,Cyclo(2/27)-des-AA⁷⁻²⁴[Glu²,Gly⁶,DDpr²⁷]NPY, Dicyclo(2/27,28/32)-des-AA⁷⁻²⁴[Glu^{2,32},DAla⁶,DDpr²⁷, Lys²⁸]NPY, [Ala(31),Aib(32)]-NPY,[Ala(31) oder Pro(32)]-NPY.

15. Der Y-Rezeptor zur Verwendung gemäß Anspruch 14, wobei Y ein Y2-Rezeptor, ein Y5-Rezeptor oder ein Y2/Y5-Rezeptor-Heterodimer ist.

## Revendications

1. Utilisation d'un agoniste de récepteur Y dans la préparation d'un médicament destiné à accroitre la stabilité d'une greffe de graisse, le médicament étant adapté à une administration à la greffe de graisse et/ou à un receveur de greffe de graisse dans ou à proximité du site de la greffe, l'agoniste de récepteur Y étant un ou plusieurs des composés suivants : NPY, NPY₂₋₃₆, NPY₃₋₃₆, NPY₁₃₋₃₆, NPY18-36 ou [Leu³¹, Pro³⁴]NPY, PYY₃₋₃₆, C2-NPY, [Ala³]NPY, [Ala³⁰]NPY, [Ala⁵]NPY, [Ala¹³]NPY, [Ala²⁰]NPY, [Ala²¹] NPY, [Ala²²] NPY, [Ala⁸]NPY, [Ala²⁷]NPY, [Ala²⁸]NPY, [Ala³⁶]NPY, TASP-V, N-acétyl [Leu^{28,31}]NPY²⁴⁻³⁶, TyrIleAsnLeuIleTyrArgLeuArgTyr-NH₂, Ac[Leu^{28,31}]NPY²⁴⁻³⁶, Ac-cyclo^{28/32}[Ala²⁴, Lys²⁸, Leu³¹, Glu³² ]NPY²⁴⁻³⁶, Ac-cylo^{28/32}- [Ala²⁴, Lys²⁸, Glu³²]NPY²⁴⁻³⁶, Ac-Cyclo^{28/32}[Lys²⁸, Glu³²]NPY²⁵⁻³⁶, Cyclo(2/27)-des-AA⁷⁻²⁴-[Glu², Gly⁶, DDpr²⁷]NPY, Dicyclo(2/27,28/32)-des-AA⁷⁻²⁴-[Glu^{2,32}, DAla⁶, DDpr²⁷, Lys²⁸]NPY, [Ala(31), Aib(32)]-NPY, [Ala(31) ou Pro(32)]-NPY.

2. Utilisation selon la revendication 1, dans laquelle le récepteur Y est un récepteur Y2 ou un récepteur Y5, ou un récepteur hétérodimère Y2/Y5.

3. Utilisation selon l'une quelconque des revendications précédentes, dans laquelle le médicament comprenant l'agoniste de récepteur Y est formulé dans une formule à libération prolongée ou dans un implant.

4. Utilisation selon l'une quelconque des revendications précédentes, dans laquelle le médicament comprenant l'agoniste de récepteur Y est adapté à une administration par injection, en étant administré par voie transcutanée et délivré sous la forme d'un médicament isolé ou dans une composition pharmaceutique.

5. Utilisation selon l'une quelconque des revendications précédentes, dans laquelle l'agoniste de récepteur Y est administré au moins deux fois.

6. Utilisation selon l'une quelconque des revendications précédentes, dans laquelle la greffe de graisse est une greffe autologue ou une xénogreffe.

7. Utilisation selon l'une quelconque des revendications précédentes, dans laquelle la greffe de graisse est obtenue par excision directe, liposuccion ou lipoaspiration.

8. Utilisation selon l'une quelconque des revendications précédentes, dans laquelle la greffe de graisse est un tissu adipeux.

9. Utilisation selon l'une quelconque des revendications précédentes, comprenant en outre l'administration d'un agoniste de récepteur Y à la greffe de graisse avant son implantation dans le receveur de la greffe de graisse.

10. Utilisation selon l'une quelconque des revendications précédentes, dans laquelle la greffe de graisse est obtenue à partir d'un dépôt sous-cutané ou d'un dépôt viscéral.

11. Utilisation selon l'une quelconque des revendications précédentes, dans laquelle la greffe est implantée entre l'épiderme et les plans tissulaires d'une enveloppe musculaire, intramusculaires, sus-périostés et sous-périostés, ou dans un sein, une lèvre, une joue, le visage ou le front du receveur de la greffe de graisse.

12. Utilisation selon l'une quelconque des revendications précédentes, dans laquelle le médicament est adapté à un usage dans les domaines de l'augmentation mammaire, de la reconstruction faciale, de la reconstruction mammaire, de la révision d'une liposuccion, du traitement de l'émaciation faciale due aux inhibiteurs de protéases du VIH ; de la chirurgie reconstructrice ou de la chirurgie esthétique.

13. Utilisation selon l'une quelconque des revendications précédentes, dans laquelle l'agoniste de récepteur Y est NPY ou PYY.

14. Agoniste de récepteur Y utilisable pour accroitre la stabilité d'une greffe de graisse chez un individu, dans lequel l'agoniste de récepteur Y est un ou plusieurs des composés suivants : NPY, NPY₂₋₃₆, NPY₃₋₃₆, NPY₁₃₋₃₆, NPY18-36 ou [Leu³¹, Pro³⁴]NPY, PYY₃₋₃₆, C2-NPY, [Ala³]NPY, [Ala³⁰]NPY, [Ala⁵]NPY, [Ala¹³]NPY, [Ala²⁰]NPY, [Ala²¹] NPY, [Ala²²] NPY, [Ala⁸]NPY, [Ala²⁷] NPY, [Ala²⁸] NPY, [Ala³⁶]NPY, TASP-V, N-acétyl[Leu^{28,31}]NPY²⁴⁻³⁶, TyrIleAsnLeuIleTyrArgLeuArgTyr-NH₂, Ac[Leu^{28,31}] NPY²⁴⁻³⁶, Ac-cyclo^{28/32}[Ala²⁴, Lys²⁸, Leu³¹, Glu³²]NPY²⁴⁻³⁶, Ac-cyclo^{28/32}-[Ala²⁴, Lys²⁸, Glu³²]NPY²⁴⁻³⁶, Ac-Cyclo^{28/32}[Lys²⁸, Glu³²]NPY²⁵⁻³⁶, Cyclo(2/27)-des-AA⁷⁻²⁴- [Glu², Gly⁶, DDpr²⁷]NPY, Dicyclo(2/27,28/32)-des-AA⁷⁻²⁴-[Glu^{2,32}, DAla⁶, DDpr²⁷, Lys²⁸]NPY, [Ala(31), Aib(32)]-NPY, [Ala(31) ou Pro(32)]-NPY.

15. Récepteur Y pour un usage selon la revendication 14, dans lequel le récepteur Y est un récepteur Y2, un récepteur Y5 ou un récepteur hétérodimère Y2/Y5.


### SEQUENCE LISTING

<110> Georgetown University
<120> compositions and Methods for Lipo Modeling
<130> PP824EP
<140> 06772251.2
   <141> 2006-06-06
<150> 60/688271
   <151> 2005-06-06
<160> 8
<170> PatentIn version 3.5
<210> 1
   <211> 25
   <212> DNA
   <213> Artificial Sequence
<220>
   <223> NPY primer
<400> 1
   ccagaactcg gcttgaagac cctcc 25
<210> 2
   <211> 25
   <212> DNA
   <213> Artificial Sequence
<220>
   <223> DPPIV primer
<400> 2
   tgaagcagcc agacaatttt caaaa 25
<210> 3
   <211> 25
   <212> DNA
   <213> Artificial Sequence
<220>
   <223> Y1 primer
<400> 3
   tccaaagagg attgttcagt tcaag 25
<210> 4
   <211> 25
   <212> UNA
   <213> Artificial Sequence
<220>
   <223> Y2 primer
<400> 4
   aggtccaggt cagttgtaga ctctt 25
<210> 5
   <211> 25
   <212> DNA
   <213> Artificial sequence
<220>
   <223> Y5 primer
<400> 5
   aagaaaggat tgattcaaga aagac 25
<210> 6
   <211> 25
   <212> DNA
   <213> Artificial Sequence
<220>
   <223> [beta]-actin primer
<400> 6
   atgccccccc catgccatcc tgcgt 25
<210> 7
   <211> 20
   <212> DNA
   <213> Artificial sequence
<220>
   <223> Y2 gene
<400> 7
   cctctgcacc taatgggccc 20
<210> 8
   <211> 20
   <212> DNA
   <213> Artificial sequence
<220>
   <223> Y2 gene antisense
<400> 8
   ccatggtaat ccgccgctcc 20